# EUROPÄISCHE PATENTSCHRIFT

(11) **EP 3 213 707 B1**
(45) Veröffentlichungstag und Bekanntmachung des Hinweises auf die Patenterteilung: **03.10.2018**
(21) Anmeldenummer: 17158750.4
(22) Anmeldetag: 01.03.2017
(51) Int. Cl.: A61B 17/88, B05C 17/01, B01F 5/00, B01F 3/10

(54) **LAGERUNGS- UND MISCHSYSTEM FÜR PASTENFÖRMIGE ZEMENTKOMPONENTEN UND VERFAHREN DAFÜR**
STORAGE AND MIXING SYSTEM FOR PASTY CEMENT COMPONENTS AND METHOD THEREFOR
SYSTÈME MÉLANGEUR ET DE STOCKAGE DE COMPOSANTS DE CIMENT SOUS FORME DE PÂTE ET PROCÉDÉ ASSOCIÉ

(30) Priorität: 03.03.2016 DE 102016103816
(43) Veröffentlichungstag der Anmeldung: 06.09.2017
(73) Patentinhaber: Heraeus Medical GmbH, 61273 Wehrheim (DE)
(72) Erfinder: VOGT, Sebastian, 99092 Erfurt (DE); KLUGE, Thomas, 56179 Vallendar (DE)
(74) Vertreter: Schultheiss & Sterzel Patentanwälte PartG mbB

(56) Entgegenhaltungen:
- EP-A2- 0 119 847
- EP-B1- 1 392 450
- DE-A1- 2 521 392
- DE-A1- 10 337 790

## Beschreibung

Die Erfindung betrifft eine Lagerungs- und Mischsystem für pastenförmige Zweikomponenten-Polymethylmethacrylat-Knochenzemente, das Lagerungs- und Mischsystem aufweisend eine röhrenförmige Kartusche mit einem zylindrischen Innenraum und einen axial in dem Innenraum der Kartusche verschiebbaren Austragskolben.

Die Erfindung betrifft auch ein Verfahren zur Vermischung von pastenförmigen Zementkomponenten eines Zementteigs, insbesondere eines pastenförmigen Polymethylmethacrylat-Knochenzements, mit einem solchen Lagerungs- und Mischsystem.

Gegenstand der Erfindung ist somit ein einfaches, kostengünstig herzustellendes Lagerungs- und Mischsystem für pastenförmigen Zweikomponenten-Polymethylmethacrylat-Knochenzement, mit dem die hochviskosen, pastenförmigen Komponenten des Polymethylmethacrylat-Knochenzements mit manuell bedienbaren Austragsvorrichtungen gemischt und ausgetragen werden können.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeitszementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Austragsvorrichtung beim Auspressvorgang angreift, eine Fläche von 7,0 cm² bis 12,5 cm². Die Bewegung des Austragskolbens wird durch manuell bedienbare, mechanische Austragsvorrichtungen bewirkt, die auch als Applikatoren bezeichnet werden. Diese Austragsvorrichtungen beziehungsweise Applikatoren haben normalerweise eine Auspresskraft im Bereich von ungefähr 1,5 kN bis 3,5 N.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Zementkomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Zementkomponenten wird nach der Vermischung der beiden Zementkomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden.

Eigene Versuche im Rahmen der vorliegenden Erfindung zeigten, dass während des Auspressvorgangs der Kartuschen auf Grund der hohen Viskosität der pastenförmigen Zementkomponenten ein sehr großer Druckabfall am statischen Mischer im Austragsrohr stattfindet. Eigene Versuche zeigten weiterhin, dass bei einem konischen Austragsrohr und einer Gesamtlänge von ca. 17 cm und mit einem Innendurchmesser von 11 mm am Kartuschenkopf und unter Verwendung von zehn statischen Mischelementen eine Auspresskraft größer 7 kN notwendig ist, um die hochviskosen Zementpasten in einer für den medizinischen Anwender akzeptablen Austragsgeschwindigkeit auszupressen.

Bei der Applikation der bisherigen, konventionellen PMMA-Knochenzemente, die aus einer flüssigen Monomerkomponente und einer separat dazu gelagerten Zementpulverkomponente bestehen, wird nach der Vermischung der beiden Zementkomponenten in Zementiersystemen beziehungsweise Vakuumzementiersystemen der gebildete Zementteig mit Hilfe von manuell bedienbaren Austragsvorrichtungen ausgepresst. Diese einfachen mechanischen Austragsvorrichtungen nutzen insbesondere Klemmstangen zum Auspressen, die durch einen manuell zu betätigenden Kipphebel angetrieben werden. Die manuell angetriebenen Austragsvorrichtungen sind seit Jahrzehnten weltweit erprobt und stellen bisher den Stand der Technik dar. Vorteilhaft an diesen Austragsvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Zementkomponenten unter Verwendung von Kartuschen, bei denen die Austragskolben an der äußeren Kolbenseiten, an denen die Stößel der Austragsvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Es sind auch aus DE 25 21 392 A1 und EP 0 119 847 A2 Lagerungs- und Mischsysteme für pastenförmige Zweikomponenten-Zemente bekannt, die eine röhrenförmige Kartusche mit einem Austragskolben umfassen, wobei die Kartusche eine axial angeordnete Trennwand aufweist, die den Innenraum der Kartusche in zwei getrennte Hohlräume teilt, wobei im ersten Hohlraum eine erste Zementkomponente enthalten ist und im zweiten eine zweite Zementkomponente enthalten ist, derart, dass bei der Betätigung des Kolbens, die Trennwand zerstört wird, und deshalb die zwei Komponenten miteinander gemischt werden.

Die Aufgabe der Erfindung besteht also darin, die Nachteile des Stands der Technik zu überwinden. Insbesondere soll ein einfaches, kostengünstig zu fertigendes Lagerungs- und Mischsystem für pastenförmige Zweikomponenten-Polymethylmethacrylat-Knochenzemente und ein Verfahren zur Herstellung eines Zementteigs mit einem Lagerungs- und Mischsystem bereitgestellt werden, wobei das Lagerungs- und Mischsystem als einmalig zu verwendendes ready-to-use-System in einfachster Weise mit einer minimalen Anzahl von Montageschritten innerhalb weniger Sekunden einsatzbereit ist und nach Verbindung mit manuell anzutreibenden medizinischen Austragsvorrichtungen beziehungsweise Applikatoren sofort nach Beginn der manueller Betätigung der Austragsvorrichtung einen homogen gemischten Zementteig erzeugt und an der Austragsöffnung eines Austragsrohrs austrägt. Die in den OPs bisher für die konventionellen Polymethylmethacrylat-Knochenzemente genutzten manuell zu bedienenden Austragsvorrichtungen mit jeweils einer Schubstange und einem Teller sollen für den Austrag des Zweikomponenten-Polymethylmethacrylat-Knochenzements beziehungsweise des Zementteigs mit dem zu entwickelnden Lagerungs- und Mischsystem genutzt werden. Dadurch soll die Beschaffung von speziellen Austragsvorrichtungen für den Austrag von pastenförmigen Zweikomponenten-Polymethylmethacrylat-Knochenzementen vermieden werden.

Bevorzugt soll das zu entwickelnde Lagerungs- und Mischsystem keine zwei miteinander verbundenen synchron vorzutreibende Schubstangen erfordern, damit die gesamte Vorrichtung nicht wesentlich länger und größer wird als die bisher für die konventionellen Pulver-Flüssigkeits-Polymethylmethacrylat-Knochenzemente üblichen Mischsysteme und Vakuummischsysteme. Es soll eine einfache Lösung gefunden werden, die es erlaubt möglichst mit nur einer Schubstange und einem daran befestigten Teller zwei pastenförmige Zementkomponenten synchron und manuell aus der Vorrichtung auszutreiben. Die pastenförmigen Zementkomponenten des Knochenzements sollen getrennt innerhalb des Lagerungs- und Mischsystems sicher gelagert werden. Zur Anwendung sollen beide pastenförmigen Zementkomponenten sicher zusammengeführt werden können. Das Lagerungs- und Mischsystem soll auch ein geringes Volumen des homogen gemischten Zementteigs von ca. 50 ml beziehungsweise maximal 70 ml austragen können, ohne dass größere Restmengen (mehr als 10 ml) in dem System verbleiben und aufwendig entsorgt werden müssen. Größere Volumina des Zementteigs werden nicht angestrebt. Die genannten geringen Mengen sind nämlich für viele Anwendungen ausreichend, wie beispielsweise Operationen (OPs) im Bereich des Knies.

Der Übergang von der Kartusche zum Austragsrohr soll konstruktiv möglichst so gestaltet sein, dass der Strömungswiderstand der pastenförmigen Zementkomponenten beim Auspressen möglichst niedrig ist. Als Zementkomponenten müssen pastenförmige Zementkomponenten verwendet werden, die unmittelbar nach dem Auspressen anwendbar sind, bei denen also keine Zeit zum Anquellen des PMMA-Knochenzements notwendig ist. Die Vorrichtung soll so beschaffen sein, dass eine Verwechselung der relevanten Montageschritte durch den Anwender konstruktiv soweit wie möglich ausgeschlossen ist und das Lagerungs- und Mischsystem auch von weitgehend ungeschultem Personal durchführbar ist. Weiterhin soll ein Verfahren zum Vermischen der pastenförmigen Zementkomponenten und zum Austragen des homogen gemischten Zementteigs bereitgestellt werden.

Die Aufgaben der Erfindung werden gelöst durch ein Lagerungs- und Mischsystem für pastenförmige Zweikomponenten-Polymethylmethacrylat-Knochenzemente, das Lagerungs- und Mischsystem aufweisend
a) eine röhrenförmige Kartusche mit einem zylindrischen Innenraum,
b) einen axial in dem Innenraum der Kartusche verschiebbaren Austragskolben,
c) eine axial in der röhrenförmigen Kartusche angeordnete Trennwand,
d) einen Kartuschenkopf, der ein Ende der röhrenförmigen Kartusche verschließt, wobei der Kartuschenkopf eine schlitzförmige Öffnung aufweist, wobei die Trennwand durch die schlitzförmige Öffnung des Kartuschenkopfs aus dem Innenraum der Kartusche herausragt,
wobei die Trennwand den durch den Austragskolben und den Kartuschenkopf begrenzten zylindrischen Innenraum der Kartusche in zwei räumlich voneinander getrennte Hohlräume teilt, wobei in dem ersten Hohlraum eine erste pastenförmige Zementkomponente enthalten ist und in dem separaten zweiten Hohlraum eine zweite pastenförmige Zementkomponente enthalten ist, und
wobei die Trennwand durch die schlitzförmige Öffnung des Kartuschenkopfs herausziehbar ist, so dass die beiden getrennten Hohlräume nach Herausziehen der Trennwand miteinander verbunden sind.

Unter einer Zylindergeometrie beziehungsweise unter einem zylindrischen Innenraum wird erfindungsgemäß eine allgemeine Zylinderform mit beliebiger Grundform verstanden, also nicht nur Zylinder mit kreisförmiger Grundfläche. Der zylindrische Innenraum muss also keine kreisförmigen Grundflächen aufweisen. Ein Innenraum mit senkrechter Kreiszylindergeometrie ist jedoch erfindungsgemäß bevorzugt, da die Herstellung des Lagerungs- und Mischsystems dann am einfachsten, besonders kostengünstig und in der Anwendung unanfällig für Störungen ist. Beispielsweise kann eine Verklemmung des Austragskolbens nicht so leicht auftreten.

Gemäß einer bevorzugten Ausführungsform des erfindungsgemäßen Lagerungs- und Mischsystems kann ein Sicherungselement vorgesehen sein, das gelöst werden muss, bevor die Trennwand durch die schlitzförmige Öffnung aus dem Innenraum herausziehbar ist.

Bevorzugt kann vorgesehen sein, dass die beiden Hohlräume gleiche oder in etwa gleiche Volumina haben. Besonders bevorzugt weichen die Volumina der beiden Hohlräume um weniger als 20% voneinander ab.

Der pastenförmige Zweikomponenten-Polymethylmethacrylat-Knochenzement ist durch Mischen der ersten pastenförmigen Zementkomponente mit der zweiten pastenförmigen Zementkomponente herstellbar.

Bei erfindungsgemäßen Lagerungs- und Mischsystemen kann vorgesehen sein, dass der Austragskolben an dem zum Kartuschenkopf gegenüberliegenden Ende im Innenraum der Kartusche angeordnet ist.

Dadurch kann am Kartuschenkopf oder anstatt des Kartuschenkopfs ein Austragsrohr an der Kartusche befestigt werden, durch das die beiden pastösen Zementkomponenten des PMMA-Knochenzements aus dem Innenraum der Kartusche durch Vortreiben des Austragskolbens in das Austragsrohr ausgetrieben werden können.

Es kann auch vorgesehen sein, dass das Lagerungs- und Mischsystem ein Austragsrohr aufweist, an dem ein Befestigungsmittel zur Befestigung an der Kartusche vorgesehen ist, wobei vorzugsweise das Austragsrohr anstelle des Kartuschenkopfs an der Kartusche zu befestigen ist.

Das Befestigungsmittel ist bevorzugt ein Innengewinde, das auf ein Außengewinde an der Kartusche geschraubt werden kann. Besonders bevorzugt wird das Außengewinde an der Kartusche auch zur lösbaren Befestigung des Kartuschenkopfs an der Kartusche verwendet.

Im Austragsrohr ist erfindungsgemäß bevorzugt ein statischer Mischer angeordnet. Mit der Erfindung wird auch vorgeschlagen, dass im Austragsrohr ein statischer Mischer angeordnet ist und dass an der Basis des Austragsrohrs als Verbindungsmittel ein Innengewinde, ein Außengewinde, Elemente eines Bajonettverschlusses und/oder Rastelemente eines Rastverschlusses angebracht sind.

Hiermit kann das Austragsrohr dazu verwendet werden, die Zementkomponenten zu mischen und punktgenau zu applizieren. Insbesondere bei schwer zugänglichen Orten zur Applikation eines PMMA-Knochenzements ist ein längeres Austragsrohr vorteilhaft.

Bei Lagerungs- und Mischsystemen mit Austragsrohr kann vorgesehen sein, dass das Verhältnis des Durchmessers des Innenraums der Kartusche zum Innendurchmesser des Austragsrohrs kleiner als 5 zu 2 ist, wobei bevorzugt das Verhältnis des Durchmessers des Innenraums der Kartusche zum Innendurchmesser des Austragsrohrs kleiner oder gleich 2 zu 1 ist und ganz besonders bevorzugt das Verhältnis des Durchmessers des Innenraums der Kartusche zum Innendurchmesser des Austragsrohrs 8 zu 5 ist.

Hierdurch wird erreicht, dass während des Vortreibens des Austragskolbens eine ausreichende Strömungsgeschwindigkeit des PMMA-Knochenzements an der Austragsöffnung des Austragsrohrs erreicht wird.

Es kann vorgesehen sein, dass der Durchmesser des Innenraums der Kartusche kleiner oder gleich 25 mm ist, wobei bevorzugt der Durchmesser des Innenraums der Kartusche kleiner oder gleich 20 mm ist.

Es kann bei Lagerungs- und Mischsystemen mit Austragsrohr auch vorgesehen sein, dass der Durchmesser des Innenraums der Kartusche kleiner oder gleich 25 mm ist und der Innendurchmesser des Austragsrohrs kleiner oder gleich 15 mm ist, wobei bevorzugt der Durchmesser des Innenraums der Kartusche kleiner oder gleich 20 mm ist und der Innendurchmesser des Austragsrohrs kleiner oder gleich 12 mm ist. Durch den erfindungsgemäßen Aufbau der Kartusche beziehungsweise der Kartusche und des Austragsrohrs gelingt es, beide pastöse Zementkomponenten des PMMA-Knochenzements in einer einzigen Kartusche unterzubringen, die noch durch manuelle Krafteinwirkung auspressbar ist und die aber auch gleichzeitig noch mit herkömmlichen Techniken befüllt werden kann. Bei größeren Durchmessern reicht eine manuelle Krafteinwirkung nicht mehr ohne weiteres aus, um die zähflüssigen Zementkomponenten des Knochenzements aus der Kartusche zu pressen.

Mit einer Weiterbildung der Erfindung wird vorgeschlagen, dass das Verhältnis des Durchmessers des Innenraums der Kartusche zum Abstand zwischen dem Austragskolben und dem Kartuschenkopf kleiner oder gleich 1 zu 4 ist, wobei bevorzugt das Verhältnis des Durchmessers des Innenraums der Kartusche zum Abstand zwischen dem Austragskolben und dem Kartuschenkopf kleiner oder gleich 1 zu 10 ist.

Mit einer bevorzugt Weiterbildung des erfindungsgemäßen Lagerungs- und Mischsystems wird vorgeschlagen, dass die schlitzförmige Öffnung passend zum Querschnitt der Trennwand geformt ist.

Hierdurch wird erreicht, dass die an der Trennwand anhaftenden Reste der Zementkomponenten an der schlitzförmigen Öffnung abgestreift werden und nicht in die Umgebung gelangen. Dadurch wird eine Kontamination des OP-Saals mit den Zementkomponenten vermieden. Zudem wird die Trennwand so beim Herausziehen aus dem Innenraum stabil geführt.

Es kann erfindungsgemäß vorgesehen sein, dass nach Herausziehen der Trennwand die erste pastenförmige Zementkomponente und die zweite pastenförmige Zementkomponente miteinander in Kontakt stehen.

Hiermit wird erreicht, dass die pastenförmigen Zementkomponenten des PMMA-Knochenzements nach dem Herausziehen der Trennwand innerhalb des dann gemeinsamen Innenraums der Kartusche vorliegen und gemeinsam aus dem Innenraum ausgetrieben werden können, wobei die Vermischung der beiden Zementkomponenten bereits im Innenraum der Kartusche beginnen kann.

Es wird auch vorgeschlagen, dass das Lagerungs- und Mischsystem zumindest zwei Führungselemente aufweist, die in oder an der Innenwand der Kartusche parallel zur Längsachse der Kartusche angeordnet sind, wobei die Trennwand von den zumindest zwei Führungselementen geführt ist, vorzugsweise die Trennwand formschlüssig in die zumindest zwei Führungselemente greift, und/oder der Austragskolben auf der den Innenraum begrenzenden Stirnseite ein Führungselement besitzt, in das die Trennwand eingeschoben oder eingesteckt ist.

Es können auch mehr als zwei Führungselemente vorgesehen sein.

Auf diese Weise kann eine bessere Abdichtung der beiden durch die Trennwand getrennten Hohlräume erreicht werden. Zudem wird die Trennwand so beim Herausziehen geführt beziehungsweise die Anordnung der Trennwand in dem Innenraum festgelegt.

Bei Lagerungs- und Mischsystemen mit Führungselementen kann vorgesehen sein, dass axiale Aussparungen an der Innenseite der Kartusche als Führungselemente angeordnet sind und/oder dass auf der dem Kartuschenkopf zugewandten Seite des Austragskolbens eine lineare Aussparung als Führungselement angeordnet ist, wobei bevorzugt die Enden der linearen Aussparungen an den axialen Aussparungen der Kartusche miteinander fluchten.

Besonders bevorzugt können die axialen Aussparungen durch Nuten realisiert werden.

Alternativ kann vorgesehen sein, dass axiale Stege als Führungselemente auf der Innenseite der Kartusche angeordnet sind und/oder dass ein Steg auf der dem Kartuschenkopf zugewandten Seite des Austragskolbens angeordnet ist, wobei vorzugsweise die Enden des Stegs auf dem Austragskolben an den axialen Stegen der Kartusche miteinander fluchten.

Mit diesen beiden Varianten werden ein einfacher Aufbau der Führungselemente und eine ausreichend dichte Verbindung zwischen den Führungselementen beziehungsweise der Innenwand der Kartusche und der Trennwand erreicht. Dadurch können die Zementkomponenten auch über längere Zeit stabil in der Kartusche gelagert werden.

Auch wenn die Führungselemente die Zylindergeometrie brechen, so wird der Innenraum der Kartusche im Sinne der vorliegenden Erfindung immer noch als zylindrisch angesehen. Bevorzugt brechen die Führungselemente jedoch die Zylindergeometrie nicht, sondern lediglich die Rotationssymmetrie des Innenraums, indem sie als lineare Strukturen auf der Mantelfläche des zylindrischen Innenraums parallel zur Zylinderachse vorgesehen sind.

Gemäß einer bevorzugten Weiterbildung der vorliegenden Erfindung kann vorgesehen sein, dass eine axiale Bewegung des Austragskolbens in Richtung des Kartuschenkopfs durch die Trennwand blockiert ist, wenn die Trennwand den Innenraum der Kartusche räumlich in die zwei Hohlräume teilt.

Hiermit kann sichergestellt werden, dass die beiden Zementkomponenten des PMMA-Knochenzements erst nach entfernen der Trennwand aus der Kartusche ausgetrieben werden können, so dass Anwendungsfehler vermieden werden.

Es wird im Rahmen der vorliegenden Erfindung ferner vorgeschlagen, dass in dem Kartuschenkopf zwei Durchführungen vorgesehen sind, die die beiden Hohlräume mit der Umgebung des Lagerungs- und Mischsystems verbinden, wobei in den Durchführungen jeweils ein Stopfen angeordnet ist, wobei vorzugsweise die Stopfen ein Rastelement an der dem Innenraum der Kartusche zugewandten Seite des Stopfens aufweisen.

Hierdurch können die Zementkomponenten durch die Durchführungen in die Hohlräume eingefüllt werden. Nach dem Befüllen können die Durchführungen mit Hilfe der Stopfen verschlossen werden und zwar bevorzugt aufgrund der Rastelemente derart, dass sie sich nicht mehr ohne weiteres vom Kartuschenkopf lösen lassen und daher die Kartusche dicht verschließen.

Die Stopfen und die Durchführungen sind im Querschnitt vorzugsweise halbmondförmig, kreisförmig oder kreisabschnittförmig.

Zur Erhöhung der maximal möglichen Lagerungsdauer der Zementkomponenten in dem Lagerungs- und Mischsystem kann vorgesehen sein, dass die Trennwand an der Kante zur Verbindung mit der Innenwand der Kartusche mindestens eine umlaufende gummielastische Dichtung besitzt und/oder eine Verbreiterung zur großflächigeren Abdichtung mit der Innenwand der Kartusche aufweist.

Hiermit wird eine bessere Abdichtung der beiden separaten Hohlräume voneinander erreicht und damit eine vorzeitige Reaktion der Zementkomponenten während der Lagerung vermieden. Die Trennwand ist vorzugsweise plattenförmig ausgebildet.

Bevorzugt kann des Weiteren vorgesehen sein, dass in den Hohlräumen jeweils ein Befüllungskolben angeordnet ist, die axial in den Hohlräumen beweglich sind, wobei bevorzugt die Befüllungskolben über ein Rastmittel mit dem Austragskolben verbunden sind oder verbindbar sind, wobei besonders bevorzugt im Austragskolben zwei Gegenrastmittel und zumindest eine Belüftungsöffnung vorgesehen ist, durch die Luft, die zwischen den Befüllungskolben und dem Austragskolben eingeschlossen ist, aus den Hohlräumen entweichen kann.

Die Befüllungskolben können zur Befüllung der Hohlräume mit den Zementkomponenten verwendet werden. Die Zementkomponenten werden in die Hohlräume eingepresst und dabei die Befüllungskolben in Richtung des Austragskolbens gedrückt, ohne dass im Inneren der Hohlräume unerwünschte Lufteinschlüsse verbleiben, die beim Austreiben der Zementkomponenten mit dem Austragskolben aus den Hohlräumen stören würden. Die Rastmittel dienen dazu, dass sich die Befüllungskolben mit dem Austragskolben verbinden und nicht beim Herausziehen der Trennwand verkippen können und so im Innenraum der Kartusche verkanten könnten und dadurch die Bewegung des Austragskolbens blockieren. Alternativ kann hierzu auch ein geeigneter Formschluss zwischen den Befüllungskolben und dem Austragskolben vorgesehen sein. Bevorzugt wird die zumindest eine Belüftungsöffnung durch die Befüllungskolben verschlossen, wenn diese an dem Austragskolben anliegen.

Bevorzugte Ausgestaltungen der vorliegenden Erfindung können sich dadurch auszeichnen, dass die Trennwand den Innenraum der Kartusche flüssigkeitsundurchlässig trennt und dadurch die beiden Hohlräume voneinander flüssigkeitsundurchlässig getrennt sind.

Hiermit wird sichergestellt, dass die beiden Zementkomponenten auch über längere Zeit in dem Lagerungs- und Mischsystem beziehungsweise innerhalb der Kartusche gelagert werden können. Es soll dabei vermieden werden, dass die flüssige Monomerkomponente in den benachbarten Hohlraum kriecht und mit der anderen Zementkomponente reagiert.

Es kann vorgesehen sein, dass die Kartusche, der Kartuschenkopf, die Trennwand und der Austragskolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Zementkomponenten enthaltenen Chemikalien besonders gut geeignet.

Des Weiteren kann vorgesehen sein, dass der Kartuschenkopf mit einer gummielastischen Platte und einer Überwurfkappe aufgebaut ist, wobei die Überwurfkappe eine Bewegung der gummielastischen Platte von der Kartusche weg mit Hilfe eines überragenden Rands blockiert, und wobei sich die schlitzförmige Öffnung durch die gummielastische Platte erstreckt, wobei bevorzugt die schlitzförmige Öffnung die gummielastische Platte in zwei Flächen teilt, wobei besonders bevorzugt in jeder der zwei Flächen eine Durchführung angeordnet ist, die durch einen Stopfen verschlossen ist.

Hiermit wird eine gute Dichtwirkung des Lagerungs- und Mischsystems erreicht. Die Teilung der gummielastischen Platte in zwei Flächen ist nicht so verstehen, dass die gummielastische Platte zwei separate Teile aufweisen muss. Die beiden Teile können also zusammenhängend sind und durch eine einteilige gummielastische Platte realisiert sein.

Es kann dabei auch vorgesehen sein, dass der Kartuschenkopf zusätzlich mit einer Kunststoffplatte aufgebaut ist, durch die sich die schlitzförmige Öffnung erstreckt, wobei die schlitzförmige Öffnung die Kunststoffplatte in zwei Flächen teilt, wobei in jeder der zwei Flächen eine Öffnung angeordnet ist, die durch Stopfen verschlossen sind, wobei die Kunststoffplatte auf oder unter der gummielastischen Platte im Kartuschenkopf angeordnet ist.

Ferner kann vorgesehen sein, dass eine Überwurfkappe als Verbindungselement zur Verbindung des Kartuschenkopfs mit der Kartusche vorgesehen ist, wobei die Überwurfkappe ein Innengewinde oder ein Außengewinde oder ein Bajonettverschluss oder Rastelemente aufweist.

Die Überwurfkappe ist bevorzugt eine Überwurfmutter und kann auf die Kartusche aufgeschraubt werden. Die Überwurfkappe kann als Teil des Kartuschenkopfs aufgefasst werden. Hierdurch kann der Kartuschenkopf stabil mit der Kartusche verbunden werden. Mit dem Verbindungselement kann die Überwurfkappe sicher mit der Kartusche verbunden werden. Eine Ablösung der Kartuschenkopfs von der Kartusche währender Lagerung und des Transports kann dadurch sicher verhindert werden.

Mit einer Weiterbildung der vorliegenden Erfindung wird vorgeschlagen, dass die Trennwand in dem Bereich, der außerhalb des Innenraums und außerhalb des Kartuschenkopfs angeordnet ist, mindestens ein Befestigungsmittel für eine Zugvorrichtung zur Entfernung der Trennwand aus der Kartusche aufweist.

Hiermit kann die Trennwand leichter entfernt werden. Das Befestigungsmittel ist bevorzugt eine Rastzunge, die in ein Gegenrastmittel der Zugvorrichtung greift oder greifen kann. An diesem Befestigungsmittel kann als Zugvorrichtung ein T-förmiger Handgriff, eine kugelförmiger Handgriff oder auch ein bohnenförmiger Handgriff angeordnet sein oder befestigt werden.

Es kann ferner vorgesehen sein, dass die Stirnfläche der Kartusche als Kartuschenkopf ausgebildet ist, die mindestens eine schlitzförmige Öffnung besitzt, welche die Stirnfläche in zwei Flächen teilt, wobei vorzugsweise in jeder der zwei Flächen eine Öffnung angeordnet ist, die mit einem Stopfen verschlossen ist.

Zur leichteren Anwendbarkeit des Lagerungs- und Mischsystems kann vorgesehen sein, dass an dem Kartuschenkopf eine Kappe angeordnet ist, wobei das obere Ende der Kappe als Handgriff ausgebildet ist und wobei am unteren Rand der Kappe ein Verbindungselement angeordnet ist oder Rastelemente angeordnet sind, welches oder welche die Kappe reversibel lösbar mit dem Kartuschenkopf verbinden, wobei an der Innenseite der Kappe eine Befestigungselement angebracht ist, das mit einem Befestigungselement der Trennwand irreversibel verbunden oder verbindbar ist.

Hiermit kann die Trennwand mit Hilfe des Handgriffs leicht und bequem manuell aus der Kartusche entfernt beziehungsweise herausgezogen werden.

Es kann dabei vorgesehen sein, dass die Kappe hohl ist und den Kartuschenkopf umschließt.

Bevorzugt kann vorgesehen sein, dass die Kartusche an einem Ende ein Befestigungselement für eine Auspressvorrichtung aufweist und am gegenüberliegenden Ende ein Außengewinde oder ein Innengewinde oder ein Element eines Bajonettverschlusses oder ein Rastelement eines Rastverschlusses als Verbindungselement aufweist.

Die der vorliegenden Erfindung zugrundeliegenden Aufgaben werden auch gelöst durch ein Verfahren zur Vermischung von pastenförmigen Zementkomponenten eines pastenförmigen Zementteigs, insbesondere eines Polymethylmethacrylat-Knochenzements, mit einem solchen Lagerungs- und Mischsystem mit den folgenden nacheinander ablaufenden Schritten:
a) Herausziehen der Trennwand aus der Kartusche durch den Kartuschenkopf, so dass die beiden Hohlräume miteinander verbunden werden,
b) Entfernen des Kartuschenkopfs von der Kartusche oder Entfernen von zumindest zwei Stopfen aus zumindest zwei Durchführungen im Kartuschenkopf, so dass die Kartusche geöffnet wird,
c) Aufsetzen und Verbinden eines Austragsrohrs mit der geöffneten Kartusche, wobei das Austragsrohr einen Mischer enthält,
d) Einsetzen der Kartusche in einen Applikator,
e) Auspressen der pastenförmigen Zementkomponenten mit Hilfe des Applikators durch axiale Bewegung des Austragskolbens in Richtung des Austragsrohrs, wobei die beiden Zementkomponenten durch den Mischer im Austragsrohr zu dem pastenförmigen Zementteig gemischt werden und
f) Auspressen des gemischten pastenförmigen Zementteigs aus einer Austragsöffnung des Austragsrohrs.

Dabei kann vorgesehen sein, dass zur Entfernung des Kartuschenkopfs von der Kartusche in Schritt b) ein Verbindungselement gelöst wird, das den Kartuschenkopf mit der Kartusche verbindet.

Hierdurch wird eine stabilere Verbindung zwischen dem Kartuschenkopf und der Kartusche erreicht. Zudem kann das Gegenstück an der Kartusche, das heißt ein Verbindungsmittel an der Kartusche auch zur Verbindung des Austragsrohrs verwendet werden.

Des Weiteren kann vorgesehen sein, dass das Austragsrohr mit der Kartusche durch Verbindung des Verbindungselements des Austragsrohrs mit einem Verbindungsmittel der Kartusche verbunden wird.

Hierdurch kann sichergestellt werden, dass sich das Austragsrohr beim Auspressen des Zementteigs nicht von der Kartusche löst.

Es wird ferner vorgeschlagen, dass zum Auspressen der Zementkomponenten aus der Kartusche in das Austragsrohr mit dem Applikator eine Stange mit einem Teller als Teile des Applikators angetrieben wird, wobei der Teller auf den Austragskolben des Lagerungs- und Mischsystems drückt.

Mit derartigen Applikatoren kann der Zementteig manuell ausgetragen werden. Zudem sind solche Applikatoren einfach im Aufbau und kostengünstig.

Bei einer Weiterbildung des erfindungsgemäßen Verfahrens kann vorgesehen sein, dass die Trennwand mit einer Kappe verbunden ist und in Schritt a) die mit der Kappe verbundene Trennwand durch Ziehen der Kappe oder eines Handgriffs der Kappe vollständig aus der Kartusche nach außen gezogen wird.

Mit der Kappe kann die Trennwand einfach manuell aus der Kartusche gezogen werden.

Schließlich kann auch vorgesehen sein, dass der Applikator manuell antreibbar ist oder durch Druckluft oder elektrisch antreibbar ist.

Manuell antreibbare Applikatoren sind erfindungsgemäß bevorzugt, da sie weder an eine Druckluftquelle oder eine Energiequelle angeschlossen werden müssen, noch eine solche enthalten müssen.

Der Erfindung liegt die überraschende Erkenntnis zugrunde, dass es durch eine einfache herausziehbare Trennwand gelingt, einen zylindrischen Innenraum einer Kartusche in zwei Hohlräume zu teilen, die zur Lagerung von pastösen Zementkomponenten eines PMMA-Knochenzements geeignet sind. Ebenso überraschend wurde gefunden, dass die Zeit, bevor zwei pastenförmige Zementkomponenten in dem derart verbundenen Innenraum der Kartusche miteinander reagieren und aushärten und somit das Lagerungs- und Mischsystem unbrauchbar machen, dazu ausreicht, den Kartuschenkopf durch ein Austragsrohr zu ersetzen und das Lagerungs- und Mischsystem in einen Applikator einzusetzen, um dann den PMMA-Knochenzement auszupressen und zu applizieren. Ferner wurde überraschend gefunden, dass auf diese Weise eine schmale Kartusche mit nur einem einzigen Austragskolben zum Vortreiben der beiden Zementkomponenten verwendet werden kann. Dadurch kann die Kraft, die notwendig ist, um die Zementkomponenten zu mischen und auszutreiben, minimiert werden, so dass ein mit manueller Kraft anzutreibender Applikator zusammen mit dem Lagerungs- und Mischsystem eingesetzt werden kann, um die Zementkomponenten aus der Kartusche auszutreiben und miteinander zu mischen.

Die Erfindung basiert auf der Idee, zur Minimierung des Strömungswiderstands beim Austrag nur eine zylindrische Kartusche, anstelle von mehreren side-by-side-Kartuschen oder Koaxial-Kartuschen, für die separate Lagerung der beiden pastenförmigen Zementkomponenten einzusetzen. Zur Vermeidung von zwei Schubstangen und zwei Tellern zum Antreiben von zwei Austragskolben wird die zylindrische Kartusche dazu mit einer axial herausziehbaren Trennwand ausgerüstet, die den Innenraum der Kartusche, der durch einen Austragskolben und einen Kartuschenkopf begrenzt ist, in zwei Hohlräume teilt, in denen während der Lagerung die beiden pastenförmigen Zementkomponenten separat gelagert werden können. Durch das Entfernen der Trennwand wird der Strömungswiderstand der auszupressenden pastösen Zementkomponenten derart reduziert, dass auch kleinere Mengen des PMMA-Knochenzements verwendet werden können und auch schmalere Kartuschen mit Innenräumen mit kleineren Innendurchmessern noch auspressbar sind. Unmittelbar vor der Anwendung wird die Trennwand aus der Kartusche herausgezogen. Dabei entsteht ein zylinderförmiger Hohlraum, in dem sich die beiden pastenförmigen Zementkomponenten für wenige Sekunden, bis zur Vermischung im statischen Mischer, der vor der Austragsöffnung befestigt werden kann, berühren können.

Unmittelbar nach der Entfernung der Trennwand wird in einer ersten Ausführungsform der Kartuschenkopf entfernt und das Austragsrohr, das einen statischen Mischer enthält, direkt mit einem Verbindungselement mit der Kartusche verbunden. Unmittelbar anschließend wird die Kartusche mit dem angeschlossenen Austragsrohr mit einer manuell zu betätigenden Austragsvorrichtung beziehungsweise einem manuell zu betätigenden Applikator verbunden und mit dem Austrag der Zementkomponenten beziehungsweise des Zementteigs begonnen. Diese Schritte erfordern einen Zeitaufwand von ungefähr 5 bis 10 Sekunden. Nach ungefähr 30 Sekunden kontinuierlicher Betätigung der Auspressvorrichtung beziehungsweise des Applikators ist bei einem gesamten Volumen beider pastenförmigen Zementkomponenten von maximal 60 ml der Austrag des Zementteigs also des gemischtem pastösen Zweikomponenten-PMMA-Knochenzements beendet. Es wurde mit der vorliegenden Erfindung überraschend gefunden, dass auf eine komplette Trennung der beiden Zementkomponenten wenige Sekunden vor dem Auspressvorgang und während des begonnenen Auspressvorgangs verzichtet werden kann, wenn der aus den pastenförmigen Zementkomponenten hergestellte Zementteig eine Verarbeitungszeit von mindestens drei Minuten hat. Deshalb kann eine einfache gemeinsame zylindrische Kartusche mit nur einem geringen Strömungswiderstand verwendet werden.

In einer zweiten Ausführungsform werden nach Entfernung der Trennwand zwei Stopfen am Kartuschenkopf entfernt und das Austragsrohr mit statischem Mischer mit einem Verbindungselement mit der Kartusche verbunden.

Weiterhin basiert die Erfindung auf der Beobachtung, dass hochviskoser Zementteig aus zylinderförmigen Kartuschen durch ein Austragsrohr mit statischem Mischer mit handelsüblichen manuell anzutreibenden Auspressvorrichtungen beziehungsweise Applikatoren in akzeptabler Zeit und mit akzeptablem, da manuell aufbringbarem Kraftaufwand ausgetragen werden kann, wenn der Austragskolben an seiner Stirnseite einen Durchmesser von maximal 25 mm hat.

Ein erfindungsgemäßes Lagerungs- und Mischsystem für pastenförmigen Zweikomponenten-Polymethylmethacrylat-Knochenzement ist beispielsweise zusammengesetzt aus
a) einer röhrenförmigen Kartusche,
b) zwei Führungselementen, die in oder an der Innenwand der Kartusche parallel zur Längsachse der Kartusche angeordnet sind,
c) einem axial in der röhrenförmigen Kartusche verschiebbaren Austragskolben, der auf der oberen Stirnseite ein Führungselement besitzt,
d) einer axial in der röhrenförmigen Kartusche angeordneten Trennwand, die formschlüssig in zwei Führungselemente greift, die in oder an der Innenwand der Kartusche angeordnet sind,
e) einem Kartuschenkopf, der ein Ende der röhrenförmigen Kartusche verschließt, wobei der Kartuschenkopf eine schlitzförmige Öffnung besitzt, durch welche die Trennwand über die Oberseite des Kartuschenkopfs austritt,
f) einem Austragsrohr mit einem Befestigungsmittel, zur Befestigung an der Kartusche,
g) wobei die Trennwand den aus der Kartusche, dem Austragskolben und dem Kartuschenkopf gebildeten Hohlraum in zwei getrennte Hohlräume teilt, in denen eine pastenförmige erste Zementkomponente A und separat eine pastenförmige zweite Zementkomponente B angeordnet sind, und
h) wobei durch Herausziehen der Trennwand durch die schlitzförmige Öffnung des Kartuschenkopfs nach außen die beiden Hohlräume zu einem Innenraum der Kartusche vereinigt werden, in dem sich die pastenförmige erste Zementkomponente A und die pastenförmige zweite Zementkomponente B berühren.

Dabei kann vorgesehen sein, dass axiale Aussparungen an der Innenseite der Kartusche als Führungselemente angeordnet sind.

In einer ersten Ausführungsform der beispielhaften Vorrichtung ist auf der oberen Stirnseite des Austragskolbens eine linienförmige Aussparung angeordnet, wobei die Enden der Aussparungen an den beiden Aussparungen der Kartusche aneinander liegen. In dieser Ausführungsform sind axiale Stege als Führungselemente auf der Innenseite der Kartusche angeordnet. Der Austragskolben kann Rastelemente an seiner Mantelfläche oder an seiner äußeren Stirnfläche besitzen, die eine Bewegung des Austragskolbens entgegengesetzt zum Kartuschenkopf verhindern. Dies ist wichtig, wenn die Vorrichtung mit Ethylenoxid äußerlich sterilisiert wird. Bei der Sterilisation mit Ethylenoxid wird zur Entfernung der Luft aus der Sterilisationskammer ein Vakuum angelegt. Durch den Unterdruck kann es infolge der Verdampfung von Methylmethacrylat in den beiden Zementkomponenten A und B zu einer unerwünschten Bewegung des Austragskolbens entgegengesetzt zum Kartuschenkopf kommen.

In einer zweiten Ausführungsform der beispielhaften Vorrichtung ist ein Steg auf der oberen Stirnseite des Austragskolbens angeordnet, dessen Enden mit den axialen Stegen der Kartusche aneinander liegen.

In einer Ausgestaltungsform sind die Hohlräume durch zwei halbmondförmige Hohlräume realisiert, wobei in dem ersten halbmondförmigen Hohlraum ein axial beweglicher erster halbmondförmiger Befüllungskolben mit einem Rastelement an der Kolbenrückseite und in dem zweiten halbmondförmigen Hohlraum ein axial beweglicher zweiter halbmondförmiger Befüllungskolben mit einem Rastelement an der Kolbenrückseite angeordnet ist, wobei der Austragskolben jeweils eine Belüftungsöffnung und zwei Rastelemente besitzt. Bei dieser Ausgestaltungsform sind die beiden halbmondförmigen Befüllungskolben vor der Befüllung der Kartusche direkt am Kartuschenkopf angeordnet. Beim Einpressen der pastenförmigen Zementkomponenten rücken diese die halbmondförmigen Befüllungskolben in Richtung des Austragskolbens. Bei Kontakt mit dem Austragskolben verrasten diese miteinander. Beim Herausziehen der Trennwand entsteht ein Unterdruck innerhalb der Kartusche, die halbmondförmigen Befüllungskolben werden dadurch nach vorne in Richtung Kartuschenkopf gezogen. Zur Verhinderung einer Verkippung dieser Befüllungskolben ist es vorteilhaft, wenn diese mit dem Austragskolben zuvor durch Verrastung verbunden werden.

Die Trennwand der erfindungsgemäßen Vorrichtung ist bevorzugt als Platte ausgebildet, die an der Schmalseite mindestens eine umlaufende gummielastische Dichtung besitzt.

Das bedeutet, dass in der ersten Ausführungsform die Trennwand in die beiden Aussparungen der Kartusche und der Aussparung des Austragskolbens formschlüssig eingesetzt ist, wobei die umlaufende gummielastische Dichtung der Trennwand in den Aussparungen liegt und dadurch die beiden Hohlräume und die darin enthaltenen pastenförmigen Zementkomponenten flüssigkeitsundurchlässig voneinander trennt.

In der zweiten Ausführungsform ist eine umlaufende Aussparung an der Schmalseite der Trennwand ausgebildet, die von jeweils einer parallel dazu angeordneten gummielastischen Dichtung flankiert wird. Bei dieser Ausführungsform ist die Trennwand mit einer umlaufenden Aussparung auf die Stege der Kartusche und den Steg des Austragskolbens geschoben. Die Abdichtung der Hohlräume erfolgt durch die umlaufenden gummielastischen Dichtungen.

Die pastenförmigen Zementkomponenten enthalten das sehr flüchtige, radikalisch polymerisierbare Monomer Methylmethacrylat. Für eine Lagerung der Zementkomponenten ist es daher unerlässlich, dass die Kartusche, der Kartuschenkopf, die Trennwand und der Austragskolben aus Kunststoffen gefertigt sind, die eine gute Diffusionsbarriere für Methylmethacrylat darstellen. Erfindungsgemäß ist daher bevorzugt, dass die Kartusche, der Kartuschenkopf, die Trennwand und der Austragskolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden. Es zusätzlich auch möglich, auf die nicht die Zementkomponenten berührenden Teile diffusionsdichte Metallschichten, Metall- oder Halbmetalloxidschichten oder Kunststoffschichten aufzubringen. Als Metallschichten kommen insbesondere Aluminiumschichten in Betracht. Als Halbmetalloxidschichten sind insbesondere Siliziumdioxid-Schichten geeignet.

Für die Funktion des Lagerungs- und Mischsystems ist es notwendig, dass die Kartusche an einem Ende ein Befestigungselement für eine Auspressvorrichtung besitzt und dass am gegenüberliegenden Ende mindestens ein Außengewinde und/oder ein Innengewinde und/oder mindestens eines Elements eines Bajonettverschlusses und/oder mindestens ein Rastelement eines Rastverschlusses als Verbindungselement angeordnet ist.

Es kann vorgesehen sein, dass der Kartuschenkopf aus einer gummielastischen Platte und einer aus Kunststoff gefertigten Überwurfkappe gebildet ist, wobei die Überwurfkappe die gummielastische Platte nach oben durch einen überragenden Rand blockiert, und wobei die gummielastische Platte eine schlitzförmige Öffnung aufweist, die die gummielastische Platte in zwei halbmondförmige beziehungsweise halbkreisförmige Flächen teilt, wobei in jeder halbmondförmigen Fläche eine Durchführung angeordnet ist, die durch einen Stopfen verschlossen ist.

Eine weitere beispielhafte Ausgestaltungsform ist dadurch charakterisiert, dass eine Kunststoffplatte auf oder unter der gummielastischen Platte im Kartuschenkopf angeordnet ist, wobei die Kunststoffplatte eine schlitzförmige Öffnung besitzt, welche die Kunststoffplatte in zwei halbmondförmige oder halbkreisförmige Flächen teilt, wobei in jeder halbmondförmigen Fläche eine Durchführung angeordnet ist, die durch Stopfen verschlossen sind. Die Anordnung der zusätzlichen Kunststoffplatte führt zu einer verbesserten Diffusionsbarriere gegenüber dem in den Zementkomponenten enthaltenen Methylmethacrylat.

Erfindungsgemäß ist im Austragsrohr ein statischer Mischer angeordnet. Als statische Mischer kommen alle allgemein bekannten statischen Mischer in Betracht. An der Basis des Austragsrohrs sind als Verbindungsmittel ein Innengewinde und/oder ein Außengewinde und/oder Elemente eines Bajonettverschlusses und/oder Rastelemente eines Rastverschlusses angebracht. Mit diesem Verbindungsmittel kann das Austragsrohr mit der Kartusche mechanisch stabil verbunden werden. Diese Verbindung muss stabil sein, damit der bei dem Herauspressen der pastenförmigen Zementkomponenten auftretende hohe Druck nicht zu einem Ablösen des Austragsrohrs von der Kartusche führt. Besonders vorteilhafte Verbindungsmittel sind dabei Gewinde und ganz besonders vorteilhaft sind Doppelgewinde.

In einer Ausgestaltungsvariante ist die Stirnfläche der Kartusche als Kartuschenkopf ausgebildet, die mindestens eine schlitzförmige Öffnung besitzt, welche die Stirnfläche in zwei halbmondförmige beziehungsweise halbkreisförmige Flächen teilt, wobei in jeder halbmondförmigen Fläche eine Durchführung angeordnet ist, die mit einem Stopfen verschlossen ist. Diese Durchführungen können zylinderförmig, halbmondförmig oder auch nierenförmig gestaltet sein. Entsprechend der Geometrie dieser Öffnungen sind dann auch die Stopfen im Querschnitt zylinderförmig, halbmondförmig oder nierenförmig. Besonders vorteilhaft ist es, wen die Stopfen an der Unterseite Rastelemente besitzen, damit die Stopfen sich nicht aus den Durchführungen lösen können.

In einer vorteilhaften Ausgestaltungsform der Erfindung ist über dem Kartuschenkopf eine hohle Kappe angeordnet, die den Kartuschenkopf umschließt, wobei das obere Ende der Kappe als Handgriff ausgebildet ist und wobei am unteren Rand der Kappe Rastelemente angeordnet sind, welche die Kappe reversibel lösbar mit dem Kartuschenkopf verbinden. Der besondere Vorteil dieser Kappe mit Handgriff besteht darin, dass einerseits der Kartuschenkopf vor unbeabsichtigter Demontage geschützt wird und dass zwangsweise beim Entfernen der Kappe die Trennwand mit gezogen wird. Erst nach Entfernung der Kappe mit der damit verbundenen Trennwand ist eine Entfernung des Kartuschenkopfs möglich.

Beispielhaft ist auch ein erfindungsgemäßes Verfahren zur Vermischung der beiden pastenförmigen Zementkomponenten des pastenförmigen Polymethylmethacrylat-Knochenzements mit dem erfindungsgemäßen Lagerungs- und Mischsystem, mit den folgenden nacheinander ablaufenden Schritten:
a) manuelles, vollständiges Herausziehen der Trennwand aus dem Kartuschenkopf, wobei der erste Hohlraum mit dem zweiten Hohlraum einen gemeinsamen Innenraum der Kartusche bildet, in dem die pastenförmigen Zementkomponenten in Kontakt treten,
b) Lösen der Verbindungselements, das den Kartuschenkopf mit der Kartusche verbindet, und Entfernen des Kartuschenkopfs von der Kartusche,
c) Aufsetzen des Austragsrohrs, das einen statischen Mischer enthält, auf die geöffnete Kartusche,
d) Verbinden des Austragsrohrs mit der Kartusche durch Verbindung des Verbindungsmittels des Austragsrohrs mit dem Verbindungsmittel der Kartusche,
e) Verbinden der Kartusche mit einem manuell zu betätigenden Applikator,
f) Betätigen des Applikators, wobei eine Stange mit einem Teller vorgetrieben wird, der auf den Austragskolben drückt,
g) Auspressen der beiden pastenförmigen Zementkomponenten durch axiale Bewegung des Austragskolbens in Richtung des Austragsrohrs, wobei die beiden Zementkomponenten unter Bildung eines homogenen Zementteigs durch Einwirkung des statischen Mischers im Austragsrohr gemischt werden und
h) Auspressen des homogenen, gemischten Zementteigs aus der Austragsöffnung des Austragsrohrs nach außen.

Dabei kann die mit der Verschlusskappe verbundene Trennwand durch Ziehen der Verschlusskappe vollständig aus der Kartusche nach außen gezogen werden.

In einer Variante des Verfahrens wird anstelle des manuell zu betätigenden Applikators ein durch Druckluft oder elektrischen Strom angetriebener Applikator verwendet.

Ein weiteres beispielhaftes Verfahren zur Vermischung der beiden pastenförmigen Zementkomponenten des pastenförmigen Polymethylmethacrylat-Knochenzements ist durch folgende nacheinander ablaufende Schritte gekennzeichnet,
a) vollständiges Herausziehen der Trennwand aus dem Kartuschenkopf, wobei der erste Hohlraum mit dem zweiten Hohlraum einen gemeinsamen Innenraum bildet, wo die beiden Zementkomponenten miteinander in Kontakt treten,
b) Entfernen der Stopfen aus den Durchführungen des Kartuschenkopfs,
c) Aufsetzen des Austragsrohrs, das einen statischen Mischer enthält, auf die geöffnete Kartusche,
d) Verbinden des Austragsrohrs mit der Kartusche durch Verbindung des Verbindungsmittels des Austragsrohr mit dem Verbindungsmittel der Kartusche,
e) Verbinden der Kartusche mit einem manuell zu betätigenden Applikator,
f) Betätigung des Applikators, wobei eine Stange mit einem Teller ausgetrieben wird, der auf den Austragskolben drückt,
g) Auspressen der beiden Zementkomponenten durch axiale Bewegung des Austragskolbens in Richtung des Austragsrohrs, wobei die pastenförmigen Zementkomponenten unter Bildung eines homogenen Zementteigs durch Einwirkung des statischen Mischers im Austragsrohr gemischt werden und
h) Auspressen des homogenen, gemischten Zementteigs aus der Austragsöffnung des Austragsrohrs nach außen durch Betätigen des Applikators.

Bei diesem beispielhaften Verfahren besitzt die Vorrichtung keinen separaten Kartuschenkopf. Eine Stirnseite der Kartusche ist als Kartuschenkopf ausgebildet.

Im Folgenden werden Ausführungsbeispiele der Erfindung anhand von elf schematisch dargestellten Figuren erläutert, ohne jedoch dabei die Erfindung zu beschränken. Dabei zeigt:
Figur 1: eine schematische perspektivische Ansicht auf ein erfindungsgemäßes Lagerungs- und Mischsystem;
Figur 2: eine schematische Querschnittansicht durch das erfindungsgemäße Lagerungs- und Mischsystem nach Figur 1 unmittelbar vor der Applikation des PMMA-Knochenzements, bei dem die Kappe und der Griff entfernt wurde und der Kartuschenkopf durch ein Austragsrohr ersetzt ist;
Figur 3: eine schematische perspektivische Querschnittansicht auf den vorderen Teil (in Figur 2 links) des erfindungsgemäßen Lagerungs- und Mischsystems nach Figur 2;
Figur 4: eine schematische Querschnittansicht durch das erfindungsgemäße Lagerungs- und Mischsystem nach Figur 1;
Figur 5: eine schematische Querschnittansicht durch das erfindungsgemäße Lagerungs- und Mischsystem nach Figur 1 mit einem Schnitt senkrecht zu dem nach Figur 4 gezeigten Querschnitt;
Figur 6: eine vergrößerte schematische Querschnittansicht der Vorderseite des Lagerungs- und Mischsystems gemäß der Darstellung nach Figur 4;
Figur 7: eine vergrößerte schematische perspektivische Ansicht der Vorderseite des Lagerungs- und Mischsystems nach Figur 1, 4 ,5 und 6 in einer Explosionsdarstellung;
Figur 8: mehrere schematische Darstellungen zweier unterschiedlicher Platten zum Aufbau von Kartuschenköpfen erfindungsgemäßer Lagerungs- und Mischsysteme;
Figur 9: eine vergrößerte schematische perspektivische Querschnittansicht des Kartuschenbodens eines erfindungsgemäßen Lagerungs- und Mischsystems;
Figur 10: eine vergrößerte schematische perspektivische Teilquerschnittansicht des Kartuschenbodens des erfindungsgemäßen Lagerungs- und Mischsystems nach Figur 9; und
Figur 11: eine schematische perspektivische Explosionsdarstellung mit teilweise unterschiedlichen und alternativen Teilen von erfindungsgemäßen Lagerungs- und Mischsystemen.

In den Figuren werden der Einfachheit halber für gleiche und gleichartige Bauteile unterschiedlicher Ausführungsformen teilweise die gleichen Bezugszeichen verwendet.

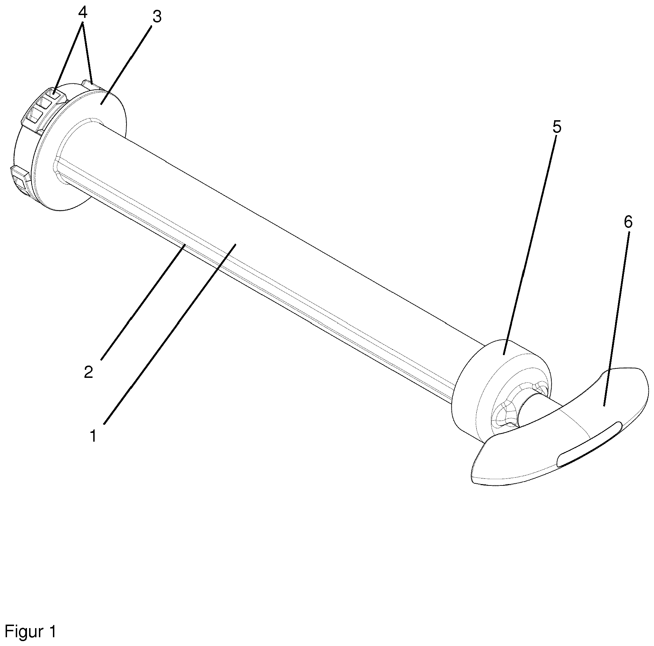

Figur 1 zeigt eine schematische perspektivische Ansicht auf ein erfindungsgemäßes Lagerungs- und Mischsystem. In Figur 1 ist der äußerlich einfache Aufbau zu erkennen. Das Lagerungs- und Mischsystem weist als zentralen Bestandteil eine zylindrische Kartusche 1 auf, bei der auf zwei gegenüberliegenden Innenseiten jeweils eine axiale Nut 2 als Führungselement vorgesehen ist. Aufgrund der gleichmäßigen Wandstärke der Kartusche 1 ist die Nut 2 in Figur 1 auch von außen als axiale Wölbung zu erkennen. Genaugenommen ist in Figur 1 also nicht die Nut 2 zu erkennen, sondern die Materialausstülpung entlang der Kartuschenwand auf der Rückseite der Nut 2. Um die axiale Lage der Nut 2 klarzustellen, wurde die Lage der Nut 2 dennoch in Figur 1 kenntlich gemacht.

An der Kartuschenunterseite beziehungsweise bodenseitig (in Figur 1 links) ist an der Kartusche 1 ein Anschluss 3 mit Befestigungselementen 4 angeordnet. Über den Anschluss 3 und die Befestigungselemente 4 kann die Kartusche 1 an eine Austragsvorrichtung beziehungsweise einen Applikator (nicht gezeigt) angeschlossen werden. An der gegenüberliegenden Vorderseite (in Figur 1 rechts) der Kartusche 1 ist eine hohle Kappe 5 aufgesetzt, die die Vorderseite beziehungsweise einen darunter angeordneten Kartuschenkopf (in Figur 1 nicht zu sehen) abdeckt. Zudem ist ein Handgriff 6 an der Vorderseite der Kartusche 1 angeordnet, der ins Innere der Kartusche 1 greift. Der Handgriff 6 ist bevorzugt mit der Kappe 5 verbunden beziehungsweise verrastet und kann als Teil der Kappe 5 aufgefasst werden.

Die Kartusche 1 hat einen Außendurchmesser von 22 mm, einen Innendurchmesser von 20 mm und eine Länge von etwa 18 cm.

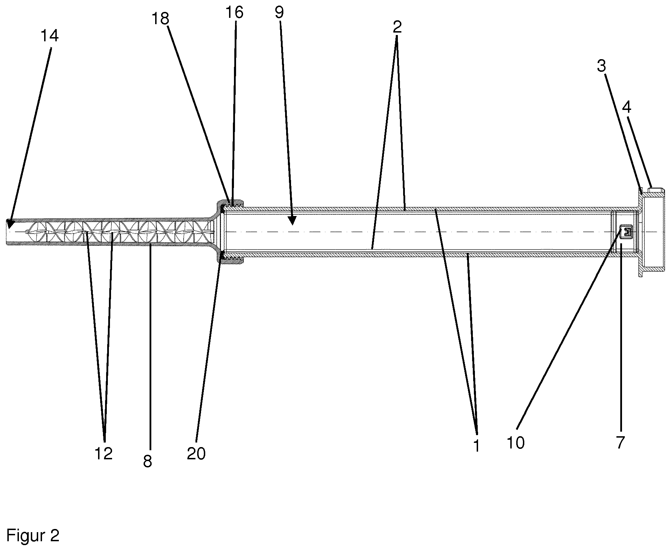

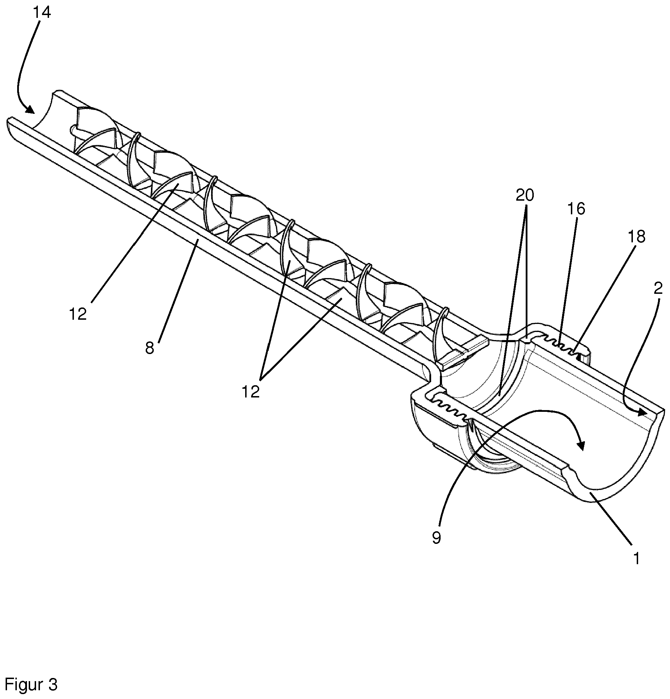

Figur 2 zeigt eine schematische Querschnittansicht durch das erfindungsgemäße Lagerungs- und Mischsystem nach Figur 1 unmittelbar vor der Applikation des PMMA-Knochenzements, bei dem die Kappe 5 und der Handgriff 6 entfernt wurden und der Kartuschenkopf durch ein Austragsrohr 8 ersetzt ist. Figur 3 zeigt eine schematische perspektivische Teil-Querschnittansicht auf den vorderen Teil (in Figur 2 links) des erfindungsgemäßen Lagerungs- und Mischsystems nach Figur 2.

Bodenseitig ist ein axial beweglicher Austragskolben 7 im Innenraum 9 der Kartusche 1 angeordnet. Der Austragskolben 7 ist mit zwei lösbaren Rastelementen 10 mit passenden Gegenrastelementen (in Form von zwei Vertiefungen) in der Innenwand der Kartusche 1 verbunden. Der Austragskolben 7 kann durch einen Druck von der Rückseite der Kartusche 1 (in Figur 2 rechts) in Richtung der Vorderseite der Kartusche 1 (in Figur 2 links) also in Richtung des Austragsrohrs 8 getrieben werden. Die Rastelemente 10 können durch einen bodenseitigen Druck auf den Austragskolben 7 ohne weiteres gelöst werden und dienen vor allem dazu, dass der Austragskolben 7 beim Befüllen des Innenraums 9 der Kartusche 1 mit den Zementkomponenten nicht bodenseitig aus der Kartusche 1 herausgedrückt werden kann, beziehungsweise nicht über die gewünschte, durch die Gegenrastelemente in der Innenwand der Kartusche 1 definierte Position hinaus in Richtung des Kartuschenbodens (in Figur 2 rechts) gedrückt werden kann.

Der Vortrieb des Austragskolbens 7 wird über einen Applikator (nicht gezeigt) erzeugt, der an den Anschluss 3 angeschlossen wird und mit dem eine Stange mit einem Teller daran manuell in Richtung des Austragsrohrs 8 vorgetrieben werden kann. Der Teller drückt dann auf den Austragskolben 7, löst dabei die Rastelemente 10 und treibt den Austragskolben 7 nach vorne in Richtung des Austragsrohrs 8. Der Austragskolben 7 schließt dicht mit den Innenwänden der Kartusche 1 ab. Der Austragskolben 7 formt also auch die Nut 2 der Kartusche 1 nach. Dadurch kann der Inhalt des Innenraums 9 der Kartusche 1, nämlich zwei in dem Innenraum 9 enthaltenen pastösen Zementkomponenten, nach vorne durch das Austragsrohr 8 ausgetrieben werden.

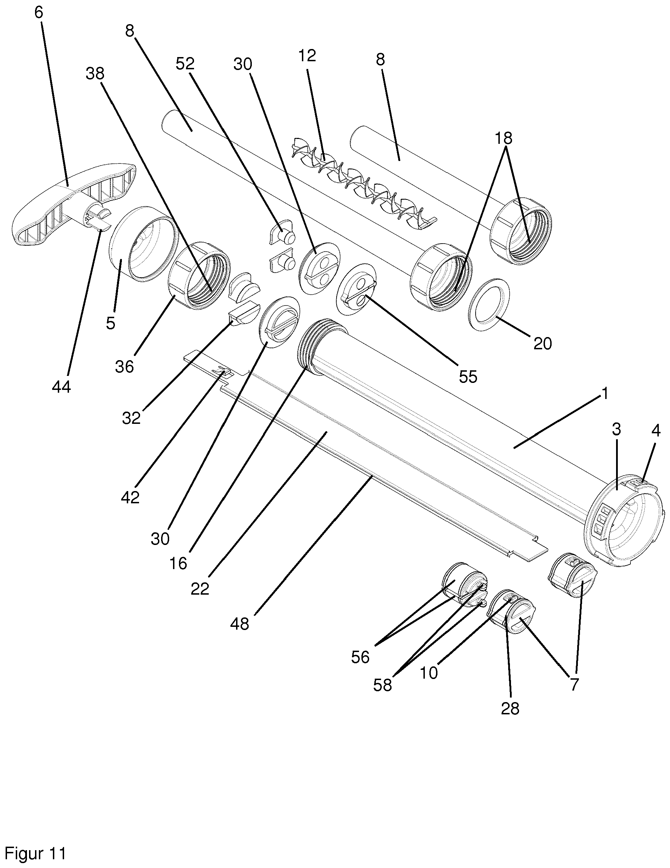

In dem Austragsrohr 8 ist ein statischer Mischer 12 enthalten, der die beiden Zementkomponenten miteinander durchmischt, bevor der derart gemischte Zementteig über eine Austragsöffnung 14 an der vorderen Spitze des Austragsrohrs 8 austritt und appliziert werden kann. Das Austragsrohr 8 kann auch noch länger sein, als das in Figur 2 gezeigte Austragsrohr 8 (siehe hierzu auch Figur 11), um schwerer zugängliche Bereiche leichter erreichbar zu machen, wie dies beispielsweise bei Operationen an der Hüfte hilfreich sein kann. In Figur 3 sind alle Teile, bis auf den statischen Mischer 12 geschnitten dargestellt, während der statische Mischer 12 perspektivisch dargestellt aus der Schnittebene herausragt. Der Schnitt nach Figur 3 ist parallel zu dem Schnitt nach Figur 2. Beide Schnitte liegen in der Ebene der beiden Nuten 2 und der Achse des Lagerungs- und Mischsystems. Die Achse des Lagerungs- und Mischsystems ist in Figur 2 durch eine Strich-Punkt-Linie gekennzeichnet. Die beiden Nuten 2 liegen also spiegelbildlich gegenüberliegend und parallel zur Achse des Lagerungs- und Mischsystems.

An der Vorderseite der Kartusche 1 ist ein Außengewinde 16 angeordnet, auf das ein Innengewinde 18 des Austragsrohrs 8 aufgeschraubt ist und so das Austragsrohr 8 auf der Vorderseite der Kartusche 1 befestigt ist. Um das Austragsrohr 8 gegen die Kartusche 1 abzudichten, ist zwischen dem vorderen Anschlag der Kartusche 1 und dem passenden Gegenstück des Austragsrohrs 8 eine umlaufende Dichtung 20 angeordnet. Mit der Dichtung 20 wird verhindert, dass Zementteig beziehungsweise die Zementkomponenten zwischen dem Austragsrohr 8 und der Kartusche 1 nach außen gedrückt werden können und so die Umgebung kontaminieren.

Das Austragsrohr 8 ist etwa 10 cm lang (kann aber auch gut 20 cm lang sein oder sogar noch länger) und hat einen Innendurchmesser von etwa 12 mm.

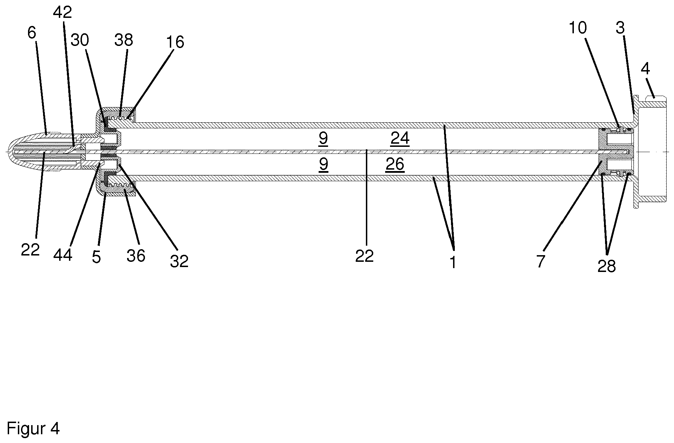

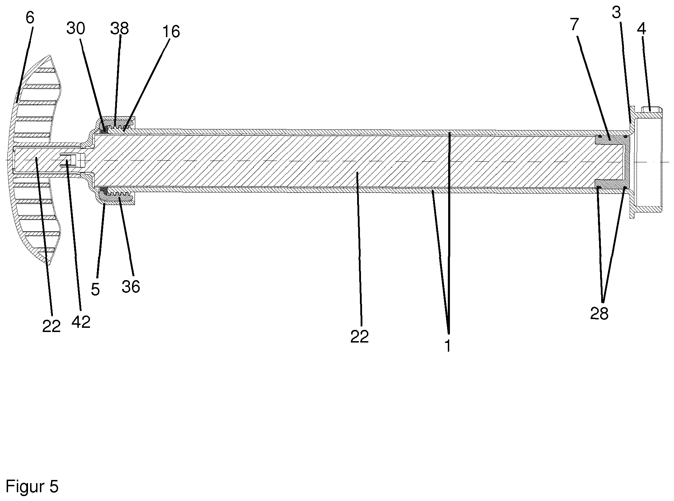

Figur 4 zeigt eine schematische Querschnittansicht durch das erfindungsgemäße Lagerungs- und Mischsystem nach Figur 1, also im Ausgangszustand. Der Schnitt durch die Kartusche 1 nach Figur 4 ist senkrecht zu dem Schnitt durch die Kartusche 1 nach Figur 2, wobei bei beiden Schnitten die Achse des Lagerungs- und Mischsystems in der Schnittebene angeordnet ist. Figur 5 zeigt eine schematische Querschnittansicht durch das erfindungsgemäße Lagerungs- und Mischsystem nach Figur 1 mit einem Schnitt senkrecht zu dem nach Figur 4 gezeigten Querschnitt, also parallel zu dem Schnitt der Kartusche 1 nach Figur 2. Auch in Figur 4 und in Figur 5 ist die Achse des Lagerungs- und Mischsystems durch eine Strich-Punkt-Linie gekennzeichnet.

Wesentlicher und zentraler Bestandteil des erfindungsgemäßen Lagerungs- und Mischsystems ist eine Trennwand 22, die sich entlang der Achse des Lagerungs- und Mischsystems erstreckt und axial aus dem Innenraum 9 der Kartusche 1 herausgezogen werden kann. Die Trennwand 22 steckt seitlich an der Innenwand der Kartusche 1 in den beiden Nuten 2, die die Trennwand 22 im Innenraum 9 der Kartusche 1 halten und positionieren. Die Trennwand 22 schließt fluiddicht an den Nuten 2 dicht und auf voller Länge mit der Innenwand der Kartusche 1 ab und trennt dadurch den Innenraum 9 der Kartusche 1 in zwei voneinander fluiddicht getrennte gleich große Hohlräume 24, 26. In dem ersten Hohlraum 24 ist die erste pastöse Zementkomponente enthalten und in dem zweiten Hohlraum 26 ist die zweite pastöse Komponente enthalten.

Die Trennwand 22 ist in einem passenden Schlitz in dem Austragskolben 7 gehalten beziehungsweise in den Schlitz eingesteckt, wobei der Schlitz im Austragskolben 7 bündig an die Nuten 2 anschließt beziehungsweise mit den Nuten 2 fluchtet und der die Trennwand 22 positioniert, hält und abdichtet. Der Austragskolben 7 ist gegen die Innenwände der Kartusche 1 mit zwei umlaufenden Dichtungen 28 abgedichtet, so dass die Zementkomponenten nicht zwischen der Kartusche 1 und dem Austragskolben 7 austreten können.

An der Vorderseite der Kartusche 1 (in den Figuren 4 und 5 links) ist eine gummielastische Platte 30 als Kartuschenkopf 30 befestigt. In dem Kartuschenkopf 30 sind zwei Durchführungen angeordnet, die einen Querschnitt in Form eines Kreisabschnitts aufweisen, beziehungsweise einen halbkreisförmigen oder halbmondförmigen Querschnitt aufweisen. In diese Durchführungen sind zwei Stopfen 32 mit passendem Querschnitt eingesetzt, so dass die Durchführungen und damit die Hohlräume 24, 26 dicht verschlossen sind. Durch die Durchführungen wären die Hohlräume 24, 26 und damit die beiden Zementkomponenten von außen zugänglich, wenn diese nicht mit den Stopfen 32 verschlossen wären.

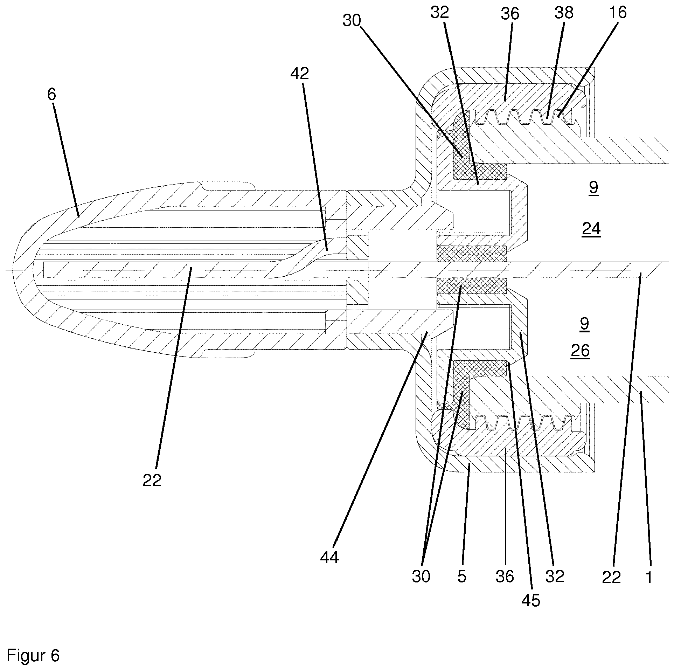

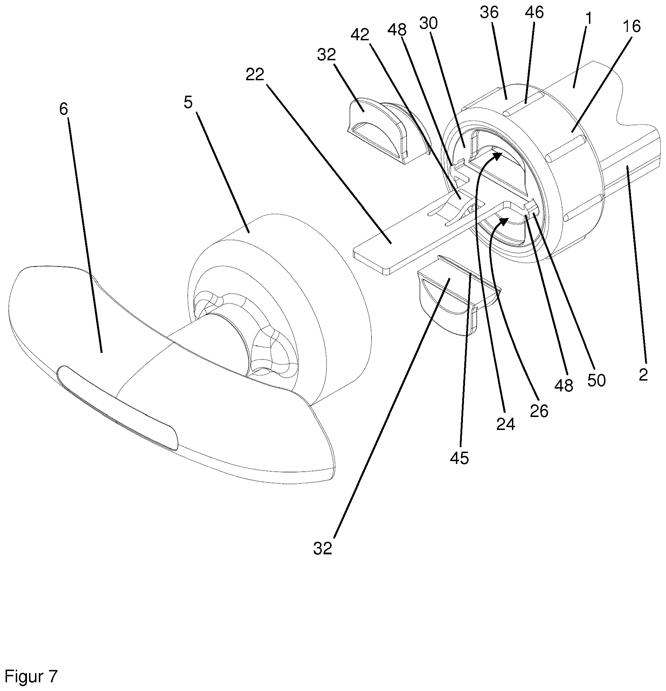

Zur besseren Übersichtlichkeit des Aufbaus des Lagerungs- und Mischsystems im Bereich von dessen Vorderseite sind vergrößerte Darstellungen in den Figuren 6 und 7 gezeigt. Dabei zeigt Figur 6 eine Ausschnittvergrößerung der Vorderseite des Lagerungs- und Mischsystems gemäß der schematische Querschnittansicht nach Figur 4 und Figur 7 eine vergrößerte schematische perspektivische Ansicht der Vorderseite des Lagerungs- und Mischsystems nach den Figuren 1, 4, 5 und 6 in einer Explosionsdarstellung.

Der Kartuschenkopf 30 mit den Stopfen 32 in den Durchführungen des Kartuschenkopfs 30 wird von einer Überwurfmutter 36 mit einem Innengewinde 38 gehalten, die auf das Außengewinde 16 der Kartusche 1 aufgeschraubt ist, auf das auch das Austragsrohr 8 aufgeschraubt wird, wenn der Kartuschenkopf 30 beziehungsweise der gesamte Aufbau auf der Vorderseite der Kartusche 1 entfernt wurde. Die Überwurfmutter 36 wird vorliegend zusammen mit der gummielastischen Platte 30 gemeinsam als Kartuschenkopf 30, 36 angesehen. Die Stopfen 32 können auch als Teil des Kartuschenkopfs 30, 36 aufgefasst werden. Durch diesen Aufbau schließt der Kartuschenkopf 30, 36 beziehungsweise die Überwurfmutter 36 und die gummielastische Platte 30 mit den Stopfen 32 in den Durchführungen der gummielastischen Platte 30 die Vorderseite des Lagerungs- und Mischsystems und die beiden Hohlräume 24, 26 dicht ab.

In dem Kartuschenkopf 30 oder genauer in der gummielastischen Platte 30 ist eine schlitzförmige Öffnung vorgesehen, durch die hindurch sich die Trennwand 22 aus dem Innenraum 9 der Kartusche 1 nach außen erstreckt. Die Trennwand 22 liegt hierzu an der schlitzförmige Öffnung an, so dass sie einerseits von der schlitzförmigen Öffnung positioniert sowie dicht gelagert wird und andererseits an der Trennwand 22 anhaftendes Material der beiden pastenförmigen Zementkomponenten an der schlitzförmigen Öffnung der gummielastischen Platte 30 abgestreift wird, wenn die Trennwand 22 durch die schlitzförmige Öffnung aus der Kartusche 1 beziehungsweise aus dem Innenraum 9 der Kartusche 1 herausgezogen wird. Die Trennwand 22 bildet im Innenraum 9 der Kartusche 1 und in der schlitzförmigen Öffnung der gummielastischen Platte 30 eine rechteckige Platte.

In dem Bereich der Trennwand 22, der außerhalb der Kartusche 1 liegt, beziehungsweise vom Innenraum 9 aus gesehen jenseits des Kartuschenkopfs 30, ist eine Rastzunge 42 vorgesehen. Mit dieser Rastzunge 42 kann die Trennwand 22 mit dem Handgriff 6 verbunden werden. Zudem sind an dem Handgriff 6 Rastungen 44 vorgesehen, die in die Kappe 5 greifen. Ebenso sind an den Stopfen 32 Rastungen 45 vorgesehen, die mit der gummielastischen Platte 30 rasten.

Die Herstellung eines erfindungsgemäßen Lagerungs- und Mischsystems kann dadurch erfindungsgemäß wie folgt ablaufen: Die Kartusche 1 mit dem Anschluss 3 daran wird bereitgestellt. Der Austragskolben 7 wird bodenseitig in die Kartusche 1 eingesetzt, bis die beiden Rastelemente 10 in die Vertiefungen in der Innenwand der Kartusche 1 greifen. Die gummielastische Platte 30 wird mit der Überwurfmutter 36 an der Vorderseite der Kartusche 1 befestigt, beziehungsweise auf das Außengewinde 16 der Kartusche 1 aufgeschraubt. Die Trennwand 22 wird durch die schlitzförmige Öffnung in der gummielastischen Platte 30 in den Innenraum 9 der Kartusche 1 eingeschoben. Die Kanten 48 der Trennwand 22 werden dabei in den Nuten 2 in den Innenwänden der Kartusche 1 geführt. Hierzu kann die Kartusche 1 senkrecht zur Achse und senkrecht zu den Nuten 2 zusammengedrückt werden, um den Abstand zwischen den Nuten 2 zu vergrößern und das Einführen der Trennwand 22 zu erleichtern. Die Trennwand 22 wird schließlich in den Schlitz in dem Austragskolben 7 eingedrückt und dadurch der Innenraum 9 der Kartusche 1 in zwei separate Hohlräume 24, 26 getrennt.

Durch die noch offenen Durchführungen in der gummielastischen Platte 30 werden die beiden pastösen Zementkomponenten getrennt voneinander in die Hohlräume 24, 26 eingefüllt und anschließend die Hohlräume 24, 26 durch einstecken der Stopfen 32 nach außen verschlossen. Die Stopfen 32 werden dazu so weit in die Durchführungen eingesteckt, bis die Rastungen 45 der Stopfen 32 mit der gummielastischen Platte 30 rasten und so nicht mehr ohne weiteres aus den Durchführungen austreten können.

Anschließend wird die Kappe 5 aufgesteckt und danach der Handgriff 6 auf die Kappe 5 aufgesteckt, wobei die Rastzunge 42 der Trennwand 22 mit dem Handgriff 6 rastet und die Rastung 44 des Handgriffs 6 mit der Kappe 5 rastet. Das Lagerungs- und Mischsystem ist damit fertig aufgebaut und kann zur Lagerung verwendet werden.

Unmittelbar vor dem Einsatz des Lagerungs- und Mischsystems wird die Trennwand 22 mit dem Handgriff 6 durch die schlitzförmige Öffnung der gummielastischen Platte 30 herausgezogen. Die beiden Zementkomponenten können nun im Innenraum 9 der Kartusche 1 miteinander in Kontakt kommen. Die Überwurfmutter 36 wird abgeschraubt und zusammen mit der gummielastischen Platte 30 und den Stopfen 32 entfernt. Stattdessen wird das Austragsrohr 8 auf die Kartusche 1 aufgeschraubt und dieser Aufbau wird in einen Applikator (nicht gezeigt) eingesetzt. Mit dem Applikator werden die Zementkomponenten aus dem nun gemeinsamen Innenraum 9 der Kartusche 1 mit Hilfe des Austragskolbens 7 in das Austragsrohr 8 gepresst, dort mit dem statischen Mischer 12 gemischt und der fertig gemischte Zementteig über die Austragsöffnung 14 appliziert.

An der Überwurfmutter 35 sind Stege 46 vorgesehen, über die die Überwurfmutter 35 von der Kappe 5, die eine glatte zylindrische Innenfläche aufweist, beabstanden. Hierdurch kann ein sterilisierendes Gas, wie beispielsweise Ethylendioxid, zwischen die Kappe 5 und die Überwurfmutter 35 vordringen und das Lagerungs- und Mischsystem für den Gebrauch im OP-Bereich leichter beziehungsweise vollständiger sterilisiert werden.

Die Kanten 48 der Trennwand 22 sind verbreitert und im Schnitt pilzförmig, um eine bessere Dichtwirkung über eine größere Anlegefläche in den dazu passenden Nuten 2 der Innenwände der Kartusche 1 zu erreichen. Zudem sind entlang der Kanten 48 gummielastische Dichtungen 50 vorgesehen, mit denen die Dichtwirkung der Trennwand 22 im Zylinder 1 verbessert werden kann und damit eine langfristigere Lagerung der Zementkomponenten in den Hohlräumen 24, 26 ermöglicht wird.

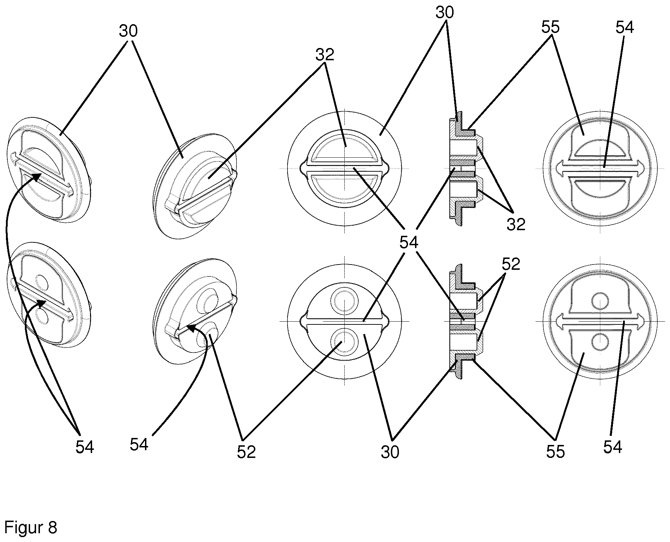

Figur 8 zeigt jeweils mehrere schematische Darstellungen zweier unterschiedlicher gummielastischer Platten 30 (erste Variante in Figur 8 oben und zweite Variante in Figur 8 unten) mit jeweils zwei unterschiedlich geformten Durchführungen. Die Varianten sind in perspektivischen Ansichten und in jeweils einer Querschnittansicht dargestellt. In den beiden Durchführungen sind jeweils ein Stopfen 32, 52 eingesteckt und gerastet. Die gummielastischen Platten 30 und die Stopfen 32, 52 sind zum Aufbau von Kartuschenköpfen erfindungsgemäßer Lagerungs- und Mischsysteme geeignet. Die gummielastischen Platten 30 unterscheiden sich in der Form ihrer Durchführungen und in der Form der Stopfen 32, 52, die diese Durchführungen verschließen. Bei der ersten Variante (Figur 8 oben) sind die Durchführungen und die Stopfen 32 im Querschnitt halbkreisförmig beziehungsweise halbmondförmig. Bei der zweiten Variante (Figur 8 unten) sind die Durchführungen und die Stopfen 52 im Querschnitt kreisförmig. Bei der ersten Variante ist der freie Querschnitt zum Einfüllen der beiden Zementkomponenten größer als bei der zweiten Variante. Dafür ist bei der zweiten Variante die Geometrie an Einfüllrohre oder Spritzen (nicht gezeigt) angepasst, über das die Zementkomponenten in die Hohlräume 24, 26 eingefüllt werden, so dass die Einfüllrohre beziehungsweise Spritzen dicht mit den Durchführungen abschließen. Die Stopfen 32, 52 können auch entfernt werden, um die Zementkomponenten wieder aus dem Innenraum 9 der Kartusche 1 auszutragen, wenn nicht der gesamte Kartuschenkopf 30, 36 entfernt werden soll.

Bei den beiden gezeigten Varianten ist zwischen den Durchführungen jeweils eine schlitzförmige Öffnung 54 in der gummielastischen Platte 30 vorgesehen, durch die die Trennwand 22 geführt ist und durch die die Trennwand 22 aus dem Innenraum 9 der Kartusche 1 herausgezogen werden kann. Bei den beiden in Figur 8 gezeigten Varianten sind an den gummielastischen Platten 30 an den in den Innenraum 9 der Kartusche 1 weisenden Seiten flache Kunststoffscheiben 55 aufgesetzt. Diese Kunststoffscheiben 55 dienen einerseits der Stabilisierung der Form der gummielastischen Platten 30 und andererseits der Verbesserung der chemischen Stabilität des Behältnisses beziehungsweise der Hohlräume 24, 26 für die Zementkomponenten.

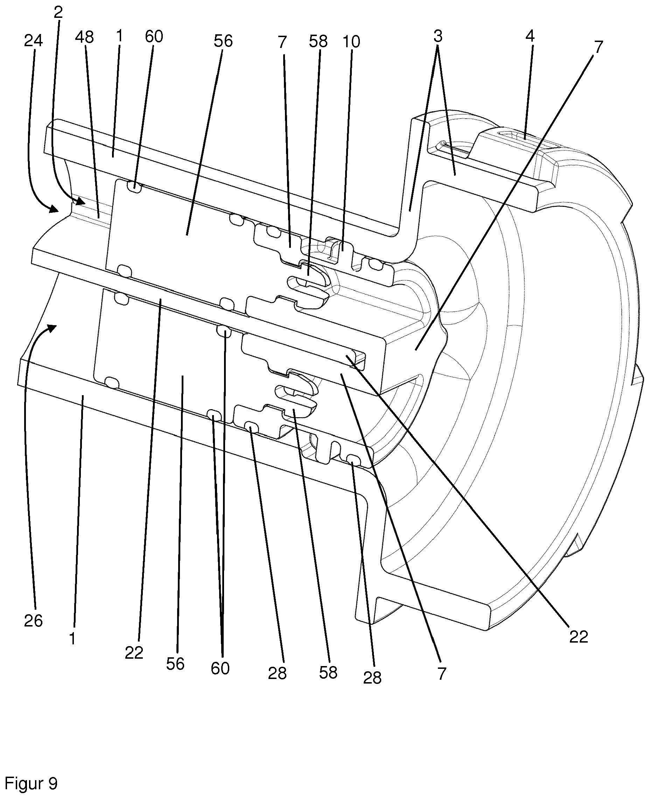

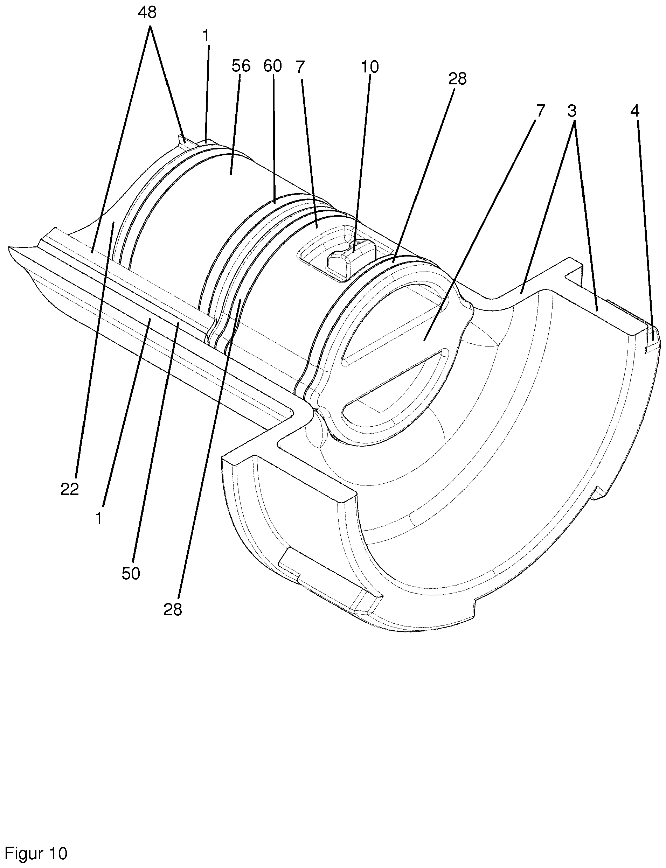

Figur 9 zeigt eine vergrößerte schematische perspektivische Querschnittansicht des Kartuschenbodens eines weiteren alternativen erfindungsgemäßen Lagerungs- und Mischsystems, bei dem das Einfüllen der Zementkomponenten erleichtert ist. Das Lagerungs- und Mischsystem ist grundsätzlich so aufgebaut, wie die mit den Figuren 1 bis 8 dargestellten Lagerungs- und Mischsysteme beziehungsweise mit den dort gezeigten Teilen. Figur 10 zeigt eine vergrößerte schematische perspektivische Teilquerschnittansicht des Kartuschenbodens des erfindungsgemäßen Lagerungs- und Mischsystems nach Figur 9.

Bei dieser Ausführungsvariante der vorliegenden Erfindung ist in den Hohlräumen 24, 26 jeweils ein Befüllungskolben 56 angeordnet. Die Befüllungskolben 56 liegen zunächst, nicht wie in Figur 9 gezeigt, an der gummielastischen Platte 30 (in Figur 9 nicht zu sehen) hinter den beiden Durchführungen in die Hohlräume 24, 26 an. Beim Einfüllen der Zementkomponenten werden die beiden Befüllungskolben 56 von den Zementkomponenten in Richtung des Austragskolbens 7 gedrückt. Dadurch kann vermieden werden, dass ein Einfüllrohr durch die Durchführungen in der gummielastischen Platte 30 bis zum Austragskolben 7 geführt werden muss, um die Zementkomponenten einzufüllen, ohne dass Lufteinschlüsse in den Hohlräumen 24, 26 entstehen. Mit dem gezeigten Lagerungs- und Mischsystem kann also eine vereinfachte Befüllung der Hohlräume 24, 26 erreicht werden, ohne dass Luft beim Einfüllen der Zementkomponenten in den pastösen Zementkomponenten innerhalb der Hohlräume 24, 26 eingeschlossen wird.

An den Befüllungskolben 56 sind Rastelemente 58 angeordnet, die in Richtung des Kartuschenbodens also in Richtung des Austragskolbens 7 weisen und die mit Öffnungen als Gegenrastmittel in dem Austragskolben 7 rasten, wenn die Befüllungskolben 56 mittels der eingefüllten Zementkomponenten auf den Austragskolben 7 gedrückt werden. Die beiden Öffnungen im Austragskolben 7 sind durchgehend, damit die Luft zwischen den Befüllungskolben 56 und dem Austragskolben 7 durch die beiden Öffnungen entweichen kann. Die Befüllungskolben 56 schließen nämlich die Hohlräume 24, 26 ab und sind hierzu mit jeweils zwei umlaufenden Dichtungen 60 gegen die Innenwand der Kartusche 1 und gegen die Trennwand 22, die die Hohlräume 24, 26 seitlich begrenzen, abgedichtet.

In Figur 9 ist gut zu erkennen, dass der Austragskolben 7 zumindest bodenseitig auch die Form der Nut 2 nachbildet. Dies ist auch bei den anderen Varianten der Fall. Bei der Variante nach Figur 9 ist dies erforderlich, da die Befüllungskolben 56 nicht in die Nut 2 eingreifen können und es nicht erwünscht ist, dass die Zementkomponenten bei Auspressen mit den Austragskolben 7 durch die Nuten 2 bodenseitig aus dem Lagerungs- und Mischsystem austreten können.

Anstatt der Rastelemente 10 mit den Vertiefungen als Gegenrastungen in den Innenwänden der Kartusche 1, kann auch die Nut 2 bodenseitig geschlossen sein oder ein Ring an der Innenwand der Kartusche 1 vorgesehen sein, der eine weitere Bewegung des Austragskolbens 7 durch den Kartuschenboden hindurch verhindert. Der Austragskolben 7 muss dann von der Vorderseite der Kartusche 1 aus in den Innenraum 9 der Kartusche 1 eingeschoben werden. Diese Ausführungsform kann für alle erfindungsgemäßen Varianten realisiert werden, ist aber in keiner der Figuren dargestellt.

Figur 11 zeigt eine schematische perspektivische Explosionsdarstellung mit teilweise unterschiedlichen und alternativen Teilen für erfindungsgemäße Lagerungs- und Mischsysteme. Der Aufbau der Lagerungs- und Mischsysteme ist analog den zuvor beschriebenen Lagerungs- und Mischsystemen.

In Figur 11 sind beispielsweise zwei unterschiedlich lange Austragsrohre 8 dargestellt, die beide mit ihren Innengewinden 18 auf das Außengewinde 16 der Kartusche 1 aufgeschraubt werden können. Das längere Austragsrohr 8 kann zum Applizieren des Zementsteigs in schwerer zugänglichen Bereichen verwendet werden. Der statische Mischer 12 und die Dichtung 20 können mit beiden Austragsrohren 8 angewendet werden.

Ebenso sind die beiden alternativen Varianten der gummielastischen Platten 30 mit unterschiedlich geformten Durchführungen dargestellt, die mit den Stopfen 32, 52 geschlossen werden können. Auch die Kunststoffscheibe 55 kann (muss aber nicht) bei der gummielastischen Platte 30 mit den im Querschnitt kreisförmigen Durchführungen eingesetzt werden.

Der Aufbau der rohrförmigen Kartusche 1 mit dem Anschluss 3, den Befestigungselementen 4, den beiden Nuten 2 und dem Außengewinde 16, ist bei allen Varianten gleich geformt. Es wäre selbstverständlich auch denkbar, nur eine Nut oder mehr als zwei Nuten vorzusehen. Die Nuten 2 könnten theoretisch schraubenförmig nach Art eines Innengewindes in oder an den Innenwänden der Kartusche 1 realisiert werden. Die Trennwand 22 müsste dann aber herausgedreht werden und auch der Austragskolben 7 müsste sich beim Vortreiben drehen, was die Realisierung des Lagerungs- und Mischsystems erschweren würde. Anstatt der Nuten 2 könnten auch Stege oder andere Erhebungen vorgesehen sein, die an der Innenwand der Kartusche 1 angeordnet sind und in denen die Trennwand 22 gehalten und abgedichtet ist.

Die Trennwand 22 ist bei allen gezeigten Ausführungsformen gleichartig. Es wäre jedoch auch möglich, die Kante 48 unterschiedlich beziehungsweise anders zu gestalten und dabei die Anlegefläche der Kante 48 an die Nut 2 und/oder die Innenwand der Kartusche 1 zu vergrößern oder zu verkleinern.

Auf das Außengewinde 16 der Kartusche 1 kann sowohl die Überwurfmutter 36 als auch das Austragsrohr 8 aufgeschraubt werden. Es sind auch Varianten denkbar, bei denen die gummielastische Platte 30 fest mit der Kartusche 1 verbunden ist oder auch nicht gummielastisch und/oder einteilig mit der Kartusche 1 ausgeführt ist. Dann wird keine Überwurfmutter 36 benötigt und das Austragsrohr 8 kann nach entfernen der Stopfen 32, 52 einfach auf die Kartusche 1 aufgeschraubt werden. Die Zementkomponenten können dann einfach durch die Durchführungen in das Austragsrohr 8 gepresst werden und dort mit dem statischen Mischer 12 zu dem gewünschten Zementteig gemischt werden.

In Figur 11 sind auch die beiden Varianten mit und ohne den Befüllungskolben 56 dargestellt. Bei der Variante ohne Befüllungskolben 56 muss der Austragskolben 7 selbstverständlich geschlossen sein, während bei der Variante mit Befüllungskolben 56 der Austragskolben 7 durchgehende Öffnungen zum Durchleiten von Luft aufweist.

Kartusche 1 und Anschluss 3 sind bei allen Varianten bevorzugt einteilig ausgeführt und bestehen bevorzugt aus Kunststoff. Bis auf die Dichtungen 20, 28, 50, 60 und die gummielastische Platte 30 können alle Teile der Lagerungs- und Mischsysteme durch Spritzguss aus Kunststoff gefertigt werden. Die Dichtungen 20, 28, 50, 60 und die gummielastische Platte 30 bestehen bevorzugt aus Gummi. Theoretisch können auch die anderen Teile der Lagerungs- und Mischsysteme aus metallischen Werkstoffen gefertigt sein. Als Zementkomponenten werden bevorzugt pastöse Ausgangskomponenten eines PMMA-Knochenzements verwendet. Theoretisch können aber auch andere Zemente, wie beispielsweise Dentalzemente, Zweikomponenten-Klebstoffe oder andere Zweikomponenten-Systeme, die aus pastösen Ausgangskomponenten gemischt werden, mit einem erfindungsgemäßen Lagerungs- und Mischsystem gelagert und gemischt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Nut / Führungselement
- 3: Anschluss
- 4: Befestigungselement
- 5: Kappe
- 6: Handgriff
- 7: Austragskolben
- 8: Austragsrohr
- 9: Innenraum
- 10: Lösbares Rastelement
- 12: Statischer Mischer
- 14: Austragsöffnung
- 16: Außengewinde
- 18: Innengewinde
- 20: Dichtung
- 22: Trennwand
- 24: Erster Hohlraum
- 26: Zweiter Hohlraum
- 28: Dichtung
- 30: Platte / Kartuschenkopf
- 32: Kolben
- 36: Überwurfmutter / Kartuschenkopf
- 38: Innengewinde
- 42: Rastzunge
- 44: Rastung
- 45: Rastung
- 46: Steg
- 48: Kante
- 50: Dichtung
- 52: Kolben
- 54: Schlitzförmige Öffnung
- 55: Kunststoffscheibe
- 56: Befüllungskolben
- 58: Rastelement
- 60: Dichtung

## Patentansprüche

1. Lagerungs- und Mischsystem für pastenförmige Zweikomponenten-Polymethylmethacrylat-Knochenzemente, das Lagerungs- und Mischsystem aufweisend
eine röhrenförmige Kartusche (1) mit einem zylindrischen Innenraum (9), einen axial in dem Innenraum (9) der Kartusche (1) verschiebbaren Austragskolben (7),
eine axial in der röhrenförmigen Kartusche (1) angeordnete Trennwand (22), einen Kartuschenkopf (30, 36), der ein Ende der röhrenförmigen Kartusche (1) verschließt, wobei der Kartuschenkopf (30, 36) eine schlitzförmige Öffnung (54) aufweist, wobei die Trennwand (22) durch die schlitzförmige Öffnung (54) des Kartuschenkopfs (30, 36) aus dem Innenraum (9) der Kartusche (1) herausragt,
wobei die Trennwand (22) den durch den Austragskolben (7) und den Kartuschenkopf (30, 36) begrenzten zylindrischen Innenraum (9) der Kartusche (1) in zwei räumlich voneinander getrennte Hohlräume (24, 26) teilt, wobei in dem ersten Hohlraum (24) eine erste pastenförmige Zementkomponente enthalten ist und in dem separaten zweiten Hohlraum (26) eine zweite pastenförmige Zementkomponente enthalten ist, und
wobei die Trennwand (22) durch die schlitzförmige Öffnung (54) des Kartuschenkopfs (30, 36) herausziehbar ist, so dass die beiden getrennten Hohlräume (24, 26) nach Herausziehen der Trennwand (22) miteinander verbunden sind.

2. Lagerungs- und Mischsystem nach Anspruch 1, **dadurch gekennzeichnet, dass**
der Austragskolben (7) an dem zum Kartuschenkopf (30, 36) gegenüberliegenden Ende im Innenraum (9) der Kartusche (1) angeordnet ist.

3. Lagerungs- und Mischsystem nach Anspruch 1 oder 2, **dadurch gekennzeichnet, dass**
das Lagerungs- und Mischsystem ein Austragsrohr (8) aufweist, an dem ein Befestigungsmittel (18) zur Befestigung an der Kartusche (1) vorgesehen ist, wobei vorzugsweise das Austragsrohr (8) anstelle des Kartuschenkopfs (30, 36) an der Kartusche (1) zu befestigen ist.

4. Lagerungs- und Mischsystem nach Anspruch 3, **dadurch gekennzeichnet, dass**
das Verhältnis des Durchmessers des Innenraums (9) der Kartusche (1) zum Innendurchmesser des Austragsrohrs (8) kleiner als 5 zu 2 ist, wobei bevorzugt das Verhältnis des Durchmessers des Innenraums (9) der Kartusche (1) zum Innendurchmesser des Austragsrohrs (8) kleiner oder gleich 2 zu 1 ist und ganz besonders bevorzugt das Verhältnis des Durchmessers des Innenraums (9) der Kartusche (1) zum Innendurchmesser des Austragsrohrs (8) 8 zu 5 ist.

5. Lagerungs- und Mischsystem nach einem der vorangehenden Ansprüche, **dadurch gekennzeichnet, dass**
der Durchmesser des Innenraums (9) der Kartusche (1) kleiner oder gleich 25 mm ist, wobei bevorzugt der Durchmesser des Innenraums (9) der Kartusche (1) kleiner oder gleich 20 mm ist.

6. Lagerungs- und Mischsystem nach einem der vorangehenden Ansprüche, **dadurch gekennzeichnet, dass**
die schlitzförmige Öffnung (54) passend zum Querschnitt der Trennwand (22) geformt ist.

7. Lagerungs- und Mischsystem nach einem der vorangehenden Ansprüche, **dadurch gekennzeichnet, dass**
das Lagerungs- und Mischsystem zumindest zwei Führungselemente (2) aufweist, die in oder an der Innenwand der Kartusche (1) parallel zur Längsachse der Kartusche (1) angeordnet sind, wobei die Trennwand (22) von den zumindest zwei Führungselementen (2) geführt ist, vorzugsweise die Trennwand (22) formschlüssig in die zumindest zwei Führungselemente (2) greift, und/oder
der Austragskolben (7) auf der den Innenraum (9) begrenzenden Stirnseite ein Führungselement (2) besitzt, in das die Trennwand (22) eingeschoben oder eingesteckt ist.

8. Lagerungs- und Mischsystem nach einem der vorangehenden Ansprüche, **dadurch gekennzeichnet, dass**
in dem Kartuschenkopf (30, 36) zwei Durchführungen vorgesehen sind, die die beiden Hohlräume (24, 26) mit der Umgebung des Lagerungs- und Mischsystems verbinden, wobei in den Durchführungen jeweils ein Stopfen (32, 52) angeordnet ist, wobei vorzugsweise die Stopfen (32, 52) ein Rastelement (45) an der dem Innenraum (9) der Kartusche (1) zugewandten Seite des Stopfens (32, 52) aufweisen.

9. Lagerungs- und Mischsystem nach einem der vorangehenden Ansprüche, **dadurch gekennzeichnet, dass**
die Trennwand (22) an der Kante (48) zur Verbindung mit der Innenwand der Kartusche (1) mindestens eine umlaufende gummielastische Dichtung (50) besitzt und/oder eine Verbreiterung zur großflächigeren Abdichtung mit der Innenwand der Kartusche (1) aufweist.

10. Lagerungs- und Mischsystem nach einem der vorangehenden Ansprüche, **dadurch gekennzeichnet, dass**
in den Hohlräumen (24, 26) jeweils ein Befüllungskolben (56) angeordnet ist, die axial in den Hohlräumen (24, 26) beweglich sind, wobei bevorzugt die Befüllungskolben (56) über ein Rastmittel (58) mit dem Austragskolben (7) verbunden sind oder verbindbar sind, wobei besonders bevorzugt im Austragskolben (7) zwei Gegenrastmittel und zumindest eine Belüftungsöffnung vorgesehen ist, durch die Luft, die zwischen den Befüllungskolben und dem Austragskolben (7) eingeschlossen ist, aus den Hohlräumen (24, 26) entweichen kann.

11. Lagerungs- und Mischsystem nach einem der vorangehenden Ansprüche, **dadurch gekennzeichnet, dass**
der Kartuschenkopf (30, 36) mit einer gummielastischen Platte (30) und einer Überwurfkappe (36) aufgebaut ist, wobei die Überwurfkappe (36) eine Bewegung der gummielastischen Platte (30) von der Kartusche (1) weg mit Hilfe eines überragenden Rands blockiert, und wobei sich die schlitzförmige Öffnung (54) durch die gummielastische Platte (30) erstreckt, wobei bevorzugt die schlitzförmige Öffnung (54) die gummielastische Platte (30) in zwei Flächen teilt, wobei besonders bevorzugt in jeder der zwei Flächen eine Durchführung angeordnet ist, die durch einen Stopfen (32, 52) verschlossen ist.

12. Lagerungs- und Mischsystem nach einem der vorangehenden Ansprüche, **dadurch gekennzeichnet, dass**
die Trennwand (22) in dem Bereich, der außerhalb des Innenraums (9) und außerhalb des Kartuschenkopfs (30, 36) angeordnet ist, mindestens ein Befestigungsmittel (42) für eine Zugvorrichtung (6) zur Entfernung der Trennwand (22) aus der Kartusche (1) aufweist.

13. Lagerungs- und Mischsystem nach einem der vorangehenden Ansprüche, **dadurch gekennzeichnet, dass**
an dem Kartuschenkopf (30, 36) eine Kappe (5) angeordnet ist, wobei das obere Ende der Kappe (5) als Handgriff (6) ausgebildet ist und wobei am unteren Rand der Kappe (5) ein Verbindungselement angeordnet ist oder Rastelemente angeordnet sind, welches oder welche die Kappe (5) reversibel lösbar mit dem Kartuschenkopf (30, 36) verbinden, wobei an der Innenseite der Kappe (5) eine Befestigungselement angebracht ist, das mit einem Befestigungselement (42) der Trennwand (22) irreversibel verbunden oder verbindbar ist.

14. Verfahren zur Vermischung von pastenförmigen Zementkomponenten eines pastenförmigen Zementteigs, insbesondere eines Polymethylmethacrylat-Knochenzements, mit einem Lagerungs- und Mischsystem nach mindestens einem der vorangehenden Ansprüche, **gekennzeichnet durch** die folgenden nacheinander ablaufenden Schritte,
a) Herausziehen der Trennwand (22) aus der Kartusche (1) durch den Kartuschenkopf (30, 36), so dass die beiden Hohlräume (24, 26) miteinander verbunden werden,
b) Entfernen des Kartuschenkopfs (30, 36) von der Kartusche (1) oder Entfernen von zumindest zwei Stopfen (32, 52) aus zumindest zwei Durchführungen im Kartuschenkopf (30, 36), so dass die Kartusche (1) geöffnet wird,
c) Aufsetzen und Verbinden eines Austragsrohrs (8) mit der geöffneten Kartusche (1), wobei das Austragsrohr (8) einen Mischer (12) enthält,
d) Einsetzen der Kartusche (1) in einen Applikator,
e) Auspressen der pastenförmigen Zementkomponenten mit Hilfe des Applikators durch axiale Bewegung des Austragskolbens (7) in Richtung des Austragsrohrs, wobei die beiden Zementkomponenten durch den Mischer (12) im Austragsrohr (8) zu dem pastenförmigen Zementteig gemischt werden und
f) Auspressen des gemischten pastenförmigen Zementteigs aus einer Austragsöffnung (14) des Austragsrohrs (8).

15. Verfahren nach Anspruch 14, **dadurch gekennzeichnet, dass** zur Entfernung des Kartuschenkopfs (30, 36) von der Kartusche (1) in Schritt b) ein Verbindungselement gelöst wird, das den Kartuschenkopf (30, 36) mit der Kartusche (1) verbindet.

16. Verfahren nach Anspruch 14 oder 15, **dadurch gekennzeichnet, dass** das Austragsrohr (8) mit der Kartusche (1) durch Verbindung des Verbindungselements (18) des Austragsrohrs (8) mit einem Verbindungsmittel (16) der Kartusche (1) verbunden wird.

17. Verfahren nach einem der Ansprüche 14 bis 16, **dadurch gekennzeichnet, dass**
die Trennwand (22) mit einer Kappe (5, 6) verbunden ist und in Schritt a) die mit der Kappe (5, 6) verbundene Trennwand (22) durch Ziehen der Kappe (5, 6) oder eines Handgriffs (6) der Kappe (5, 6) vollständig aus der Kartusche (1) nach außen gezogen wird.

## Claims

1. Storage and mixing system for pasty two-component polymethylmethacrylate bone cements, the storage and mixing system comprising
a tube-shaped cartridge (1) with a cylindrical internal space (9);
a dispensing plunger (7) that can be shifted axially in the internal space (9) of the cartridge (1);
a separating wall (22) that is arranged axially in the tube-shaped cartridge (1);
a cartridge head (30, 36) that closes one end of the tube-shaped cartridge (1), wherein the cartridge head (30, 36) comprises a slit-shaped opening (54), wherein the separating wall (22) projects through the slit-shaped opening (54) of the cartridge head (30, 36) out of the internal space (9) of the cartridge (1), wherein the separating wall (22) divides the cylindrical internal space (9) of the cartridge (1), which is bounded by the dispensing plunger (7) and the cartridge head (30, 36), into two hollow spaces (24, 26) that are spatially separate from each other,
wherein the first hollow space (24) contains a first pasty cement component and the separate second hollow space (26) contains a second pasty cement component, and
wherein the separating wall (22) can be pulled out through the slit-shaped opening (54) of the cartridge head (30, 36) such that the two separate hollow spaces (24, 26) are connected to each other after the separating wall (22) has been pulled out.

2. Storage and mixing system according to claim 1, **characterised in that** the dispensing plunger (7) is arranged in the internal space (9) of the cartridge (1), on the end opposite to the cartridge head (30, 36).

3. Storage and mixing system according to claim 1 or 2, **characterised in that** the storage and mixing system comprises a dispensing tube (8) on which an attachment means (18) for attachment to the cartridge (1) is provided, wherein the dispensing tube (8) can preferably be attached to the cartridge (1) instead of the cartridge head (30, 36).

4. Storage and mixing system according to claim 3, **characterised in that** the ratio of the diameter of the internal space (9) of the cartridge (1) and the internal diameter of the dispensing tube (8) is less than 5 to 2, wherein the ratio of the diameter of the internal space (9) of the cartridge (1) and the internal diameter of the dispensing tube (8) is preferably less than or equal to 2 to 1, and, particularly preferably, the ratio of the diameter of the internal space (9) of the cartridge (1) and the internal diameter of the dispensing tube (8) is 8 to 5.

5. Storage and mixing system according to any one of the preceding claims, **characterised in that**
the diameter of the internal space (9) of the cartridge (1) is less than or equal to 25 mm, wherein the diameter of the internal space (9) of the cartridge (1) is preferably less than or equal to 20 mm.

6. Storage and mixing system according to any one of the preceding claims, **characterised in that**
the slit-shaped opening (54) is shaped appropriately such as to match the cross-section of the separating wall (22).

7. Storage and mixing system according to any one of the preceding claims, **characterised in that**
the storage and mixing system comprises at least two guiding elements (2) that are arranged in or on the internal wall of the cartridge (1) parallel to the longitudinal axis of the cartridge (1), wherein the separating wall (22) is guided by the at least two guiding elements (2), the separating wall (22) preferably engaging with the at least two guiding elements (2) in a form-fitting manner, and/or
the dispensing plunger (7) possesses, on the front face bounding the internal space (9), a guiding element (2) into which the separating wall (22) is slid or inserted.

8. Storage and mixing system according to any one of the preceding claims, **characterised in that**
two feed-throughs that connect the two hollow spaces (24, 26) to the surroundings of the storage and mixing system are provided in the cartridge head (30, 36), wherein one stopper (32, 52) each is arranged in the feed-throughs, wherein the stoppers (32, 52) preferably comprise a snap-in element (45) on the side of the stopper (32, 52) that faces the internal space (9) of the cartridge (1).

9. Storage and mixing system according to any one of the preceding claims, **characterised in that**
the separating wall (22) possesses at least one circumferential rubber-elastic gasket (50) on the edge (48), to be connected to the internal wall of the cartridge (1), and/or a broadening for sealing against the internal wall of the cartridge (1) over a larger surface.

10. Storage and mixing system according to any one of the preceding claims, **characterised in that**
one filling plunger (56) each is arranged in the hollow spaces (24, 26), the filling plungers being axially mobile in the hollow spaces (24, 26), wherein the filling plungers (56) preferably are or can be connected to the dispensing plunger (7) by means of a snap-in means (58), wherein, particularly preferably, two opposite snap-in means and at least one ventilation opening through which air trapped between the filling plunger and the dispensing plunger (7) can escape from the hollow spaces (24, 26) are provided in the dispensing plunger (7).

11. Storage and mixing system according to any one of the preceding claims, **characterised in that**
the cartridge head (30, 36) is designed to have a rubber-elastic plate (30) and a safety cap (36), wherein the safety cap (36) blocks a motion of the rubber-elastic plate (30) away from the cartridge (1) by means of a protruding edge, and wherein the slit-shaped opening (54) extends through the rubber-elastic plate (30), wherein the slit-shaped opening (54) preferably divides the rubber-elastic plate (30) into two surfaces, wherein, particularly preferably, one feed-through that is closed by a stopper (32, 52) is arranged in each of the two surfaces.

12. Storage and mixing system according to any one of the preceding claims, **characterised in that**
the separating wall (22) comprises, in the area that is arranged outside the internal space (9) and outside the cartridge head (30, 36), at least one attachment means (42) for a pulling device (6) to remove the separating wall (22) from the cartridge (1).

13. Storage and mixing system according to any one of the preceding claims, **characterised in that**
a cap (5) is arranged on the cartridge head (30, 36), wherein the upper end of the cap (5) is formed in the way of a handle (6) and wherein a connecting element is or snap-in elements are arranged at the lower edge of the cap (5) and connecting the cap (5) to the cartridge head (30, 36) such as to be reversibly detachable, wherein an attachment element that is or can be irreversibly connected to an attachment element (42) of the separating wall (22) is secured to the internal side of the cap (5).

14. Method for mixing pasty cement components of a pasty cement dough, in particular of a polymethylmethacrylate bone cement, by means of a storage and mixing system according to at least one of the preceding claims, **characterised by** the following steps proceeding in the order given
a) pulling out the separating wall (22) from the cartridge (1) through the cartridge head (30, 36) such that the two hollow spaces (24, 26) are being connected to each other;
b) removing the cartridge head (30, 36) from the cartridge (1) or removing at least two stoppers (32, 52) from at least two feed-throughs in the cartridge head (30, 36) such that the cartridge (1) is being opened;
c) attaching and connecting a dispensing tube (8) to the opened cartridge (1), wherein the dispensing tube (8) contains a mixer (12);
d) inserting the cartridge (1) into an applicator;
e) extruding the pasty cement components by means of the applicator through axial motion of the dispensing plunger (7) in the direction of the dispensing tube, wherein the two cement components are being mixed by the mixer (12) in the dispensing tube (8) to form the pasty cement dough; and
f) extruding the mixed pasty cement dough from a dispensing opening (14) of the dispensing tube (8).

15. Method according to claim 14, **characterised in that**
for removing the cartridge head (30, 36) from the cartridge (1) in step b), a connecting element connecting the cartridge head (30, 36) to the cartridge (1) is being detached.

16. Method according to claim 14 or 15, **characterised in that** the dispensing tube (8) is being connected to the cartridge (1) by connecting the connecting element (18) of the dispensing tube (8) to a connecting means (16) of the cartridge (1).

17. Method according to anyone of the claims 14 to 16, **characterised in that** the separating wall (22) is connected to a cap (5, 6) and, in step a), the separating wall (22) connected to the cap (5, 6) is pulled fully out of the cartridge (1) toward the outside by pulling the cap (5, 6) or a handle (6) of the cap (5, 6).

## Revendications

1. Système de stockage et de mélange pour ciments osseux de polyméthacrylate de méthyle bi-composants pâteux, le système de stockage et de mélange présentant
une cartouche tubulaire (1) avec un espace intérieur (9) cylindrique, un piston d'extraction (7) déplaçable en sens axial dans l'espace intérieur (9) de la cartouche (1),
une paroi de séparation (22) disposée en sens axial dans la cartouche tubulaire (1), une tête de cartouche (30, 36) qui obture une extrémité de la cartouche tubulaire (1), la tête de cartouche (30, 36) présentant une ouverture en forme de fente (54), la paroi de séparation (22) dépassant de l'espace intérieur (9) de la cartouche (1) par l'ouverture en forme de fente (54) de la tête de cartouche (30, 36),
la paroi de séparation (22) divisant l'espace intérieur (9) cylindrique de la cartouche (1), limité par le piston d'extraction (7) et la tête de cartouche (30, 36), en deux interstices (24, 26) séparés spatialement l'un de l'autre, un premier composant de ciment pâteux étant contenu dans le premier interstice (24) et un deuxième composant de ciment pâteux étant contenu dans le deuxième interstice (26) séparé et
la paroi de séparation (22) pouvant être retirée par l'ouverture en forme de fente (54) de la tête de cartouche (30, 36) de sorte que les deux interstices (24, 26) séparés soient reliés après le retrait de la paroi de séparation (22).

2. Système de stockage et de mélange conformément à la revendication n°1, **caractérisé par le fait que**
le piston d'extraction (7) est disposé à l'extrémité opposée à la tête de cartouche (30, 36) dans l'espace intérieur (9) de la cartouche (1).

3. Système de stockage et de mélange conformément à la revendication n°1 ou n°2, **caractérisé par le fait que**
le système de stockage et de mélange présente un tuyau de sortie (8), sur lequel un moyen de fixation (18) est prévu pour une fixation sur la cartouche (1), le tuyau de sortie (8) étant de préférence à fixer sur la cartouche (1) au lieu de la tête de cartouche (30, 36).

4. Système de stockage et de mélange conformément à la revendication n°3, **caractérisé par le fait que**
le rapport entre le diamètre de l'espace intérieur (9) de la cartouche (1) et le diamètre intérieur du tuyau de sortie (8) est inférieur à 5 : 2, le rapport entre le diamètre de l'espace intérieur (9) de la cartouche (1) et le diamètre intérieur du tuyau de sortie (8) étant de préférence inférieur ou égal à 2 : 1 et, le plus préférablement, le rapport entre le diamètre de l'espace intérieur (9) de la cartouche (1) et le diamètre intérieur du tuyau de sortie (8) étant 8 : 5.

5. Système de stockage et de mélange conformément à l'une des revendications précédentes, **caractérisé par le fait que**
le diamètre de l'espace intérieur (9) de la cartouche (1) est inférieur ou égal à 25 mm, le diamètre de l'espace intérieur (9) de la cartouche (1) étant de préférence inférieur ou égal à 20 mm.

6. Système de stockage et de mélange conformément à l'une des revendications précédentes, **caractérisé par le fait que**
l'ouverture en forme de fente (54) est formée de manière à être adaptée à la coupe transversale de la paroi de séparation (22).

7. Système de stockage et de mélange conformément à l'une des revendications précédentes, **caractérisé par le fait que**
le système de stockage et de mélange présente au moins deux éléments de guidage (2) qui sont disposés dans ou sur la paroi intérieure de la cartouche (1) parallèlement à l'axe longitudinal de la cartouche (1), la paroi de séparation (22) étant guidée par les au moins deux éléments de guidage (2), la paroi de séparation (22) s'enclenchant mécaniquement de préférence dans les au moins deux éléments de guidage (2) et/ou
le piston d'extraction (7) possédant, sur la face frontale délimitant l'espace intérieur (9), un élément de guidage (2), dans lequel la paroi de séparation (22) est introduite ou insérée.

8. Système de stockage et de mélange conformément à l'une des revendications précédentes, **caractérisé par le fait que**
deux traversées qui relient les deux interstices (24, 26) à l'environnement du système de stockage et de mélange, sont prévues dans la tête de cartouche (30, 36), un bouchon (32, 52) étant disposé dans chacune des traversées, les bouchons (32, 52) présentant, de préférence, un élément d'encliquetage (45) sur la face du bouchon (32, 52) orientée vers l'espace intérieur (9) de la cartouche (1).

9. Système de stockage et de mélange conformément à l'une des revendications précédentes, **caractérisé par le fait que**
la paroi de séparation (22) possède, sur le bord (48) lié à la paroi intérieure de la cartouche (1), au moins un joint d'étanchéité (50) élastique périphérique et/ou présentant un élargissement pour une plus grande étanchéité avec la paroi intérieure de la cartouche (1).

10. Système de stockage et de mélange conformément à l'une des revendications précédentes, **caractérisé par le fait que**
un piston de remplissage (56), mobile en sens axial dans les interstices (24, 26), est disposé dans chacun des interstices (24, 26), les pistons de remplissage (56) étant de préférence reliés ou reliables avec le piston d'extraction (7) par un moyen d'encliquetage (58), deux moyens de contre-encliquetage et au moins une ouverture d'aération, par laquelle de l'air qui est enfermé entre les pistons de remplissage et le piston d'extraction (7) peut s'échapper des interstices (24, 26), étant, le plus préférablement, prévus dans le piston d'extraction (7).

11. Système de stockage et de mélange conformément à l'une des revendications précédentes, **caractérisé par le fait que**
la tête de cartouche (30, 36) est conçue avec une plaque élastique (30) et un chapeau-raccord (36), le chapeau-raccord (36) bloquant un mouvement de la plaque élastique (30) depuis la cartouche (1) à l'aide d'un bord dépassant et l'ouverture en forme de fente (54) s'étendant par la plaque élastique (30), l'ouverture en forme de fente (54) divisant de préférence la plaque élastique (30) en deux surfaces, une traversée qui est obturée par un bouchon (32, 52) étant, le plus préférablement, disposée dans chacune des deux surfaces.

12. Système de stockage et de mélange conformément à l'une des revendications précédentes, **caractérisé par le fait que**
la paroi de séparation (22) présente, dans la zone qui est disposée en dehors de l'espace intérieur (9) et en dehors de la tête de cartouche (30, 36), au moins un moyen de fixation (42) pour un dispositif de traction (6) permettant de retirer la paroi de séparation (22) de la cartouche (1).

13. Système de stockage et de mélange conformément à l'une des revendications précédentes, **caractérisé par le fait que**
un chapeau (5) est disposé sur la tête de cartouche (30, 36), l'extrémité supérieure du chapeau (5) étant formée comme poignée (6) et un élément de raccordement étant disposé ou des éléments d'encliquetage étant disposés sur le bord inférieur du chapeau (5), lequel ou lesquels relient le chapeau (5) de manière réversiblement amovible à la tête de cartouche (30, 36), un élément de fixation qui est relié ou reliable de manière irréversible à un élément de fixation (42) de la paroi de séparation (22) étant disposé sur la face intérieure du chapeau (5).

14. Procédé pour le mélange de composants de ciment pâteux d'une pâte de ciment pâteuse, notamment d'un ciment osseux de polyméthacrylate de méthyle, avec un système de stockage et de mélange conformément au moins à l'une des revendications précédentes, **caractérisé par** les étapes suivantes, se déroulant successivement,
a) Retrait de la paroi de séparation (22) de la cartouche (1) par la tête de cartouche (30, 36) de manière à ce que les deux interstices (24, 26) soient reliés,
b) Retrait de la tête de cartouche (30, 36) de la cartouche (1) ou retrait au moins de deux bouchons (32, 52) à partir au moins de deux traversées dans la tête de cartouche (30, 36) de façon à ce que la cartouche (1) s'ouvre,
c) Pose et raccordement d'un tuyau de sortie (8) avec la cartouche (1) ouverte, le tuyau de sortie (8) contenant un mélangeur (12),
d) Mise en place de la cartouche (1) dans un applicateur,
e) Pressage des composants de ciment pâteux à l'aide de l'applicateur par le mouvement axial du piston d'extraction (7) en direction du tuyau de sortie, les deux composants de ciment étant mélangés par le mélangeur (12) dans le tuyau de sortie (8) en une pâte de ciment pâteuse et
f) Pressage de la pâte de ciment pâteuse mélangée à partir d'une ouverture d'extraction (14) du tuyau de sortie (8).

15. Procédé conformément à la revendication n°14, **caractérisé par le fait que** pour retirer la tête de cartouche (30, 36) de la cartouche (1) dans l'étape b), un élément de raccordement qui relie la tête de cartouche (30, 36) à la cartouche (1) est desserré.

16. Procédé conformément à la revendication n°14 ou n°15, **caractérisé par le fait que** le tuyau de sortie (8) est relié à la cartouche (1) par le raccordement d'un élément de raccordement (18) du tuyau de sortie (8) avec un moyen de raccordement (16) de la cartouche (1).

17. Procédé conformément à la revendication n°14 à n°16, **caractérisé par le fait que**
la paroi de séparation (22) est reliée à un chapeau (5, 6) et que, dans l'étape a), la paroi de séparation (22) reliée au chapeau (5, 6) est entièrement retirée de la cartouche (1) par l'extérieur en tirant le chapeau (5, 6) ou une poignée (6) du chapeau (5, 6).
